# EUROPEAN PATENT APPLICATION

(11) **EP 1 076 066 A1**
(43) Date of publication of application: **14.02.2001**
(21) Application number: 99610043.4
(22) Date of filing: 09.08.1999
(51) Int. Cl.: C07K 14/575, A61K 38/22, A61K 47/48, A61P 3/10

(54) **Peptides for lowering blood glucose levels**

(71) Applicant: Zealand Pharmaceuticals A/S, 2600 Glostrup (DK)
(72) Inventor: Larsen, Bjarne Due, DK-2700 Broenshoej (DK); Mikkelsen, Jens Damsgaard, DK-2800 Lyngby (DK)

(57) **Abstract**

The present invention relates to novel variants that lower blood glucose levels, specific variants of exendin-4. The invention further relates to peptide conjugates that lower blood glucose levels and which have increased bioavailability.

## Description

### FIELD OF THE INVENTION

The present invention relates to novel variants that lower blood glucose levels, specific variants of exendin-4. The invention further relates to peptide conjugates that lower blood glucose levels and which have increased oral bioavailability.

### BACKGROUND OF THE INVENTION

A number of hormones that lower blood glucose levels are released from the gastrointestinal mucosa in response to the presence and absorption of nutrients in the gut. These include gastrin, secretin, glucose-dependent insulinotropic polypeptide (GIP) and glucagon-like peptide-1 (GLP-1). The most potent substance known is GLP-1 (Ørskov, 1992, Diabetologia 35:701-711).

Glucagon-like peptide 1 (GLP-1) is a product of proglucagon, a 180 amino acid peptide (Drucker, 1998, Diabetes 47:159-169). The overall sequence of proglucagon contains the 29-amino acid sequence of glucagon, the 36 or 37 amino acid sequence of GLP-1 and the 34 amino acid sequence of glucagon-like peptide-2 (GLP-2), an intestinotrophic peptide.

GLP-1 has a number of functions. It is a physiological hormone that enhances the effect on insulin secretion in normal humans and is therefore an incretin hormone. This explains in part the augmented insulin response after oral versus intravenous glucose administration. In addition, GLP-1 also lowers glucagon concentrations, slows gastric emptying, stimulates (pro)insulin biosynthesis, and enhances insulin sensitivity (Nauck, 1997, Horm. Metab. Res. 47:1253-1258). The peptide also enhances the ability for the β-cells to sense and respond to glucose in subjects with impaired glucose tolerance (Byrne, 1998, Eur. J. Clin. Invest. 28:72-78). The insulinotropic effect of GLP-1 in humans increases the rate of glucose disappearance partly because of increased insulin levels and partly because of enhanced insulin sensitivity (D'Alessio, 1994, Eur. J. Clin. Invest. 28:72-78). This has placed GLP-1 as a promising agent for treatment for type II diabetes. Active fragments of GLP-1 have been found to be GLP-1(7-36) and GLP-1(7-37). However, a major pharmacological problem with native GLP-1 is its short half-life. In humans and rats, GLP-1 is rapidly degraded by dipeptidyl peptidase-IV (DPP-IV) into GLP-1(9-36)amide, acting as an endogenous GLP-1 receptor antagonist (Deacon, 1998, Diabetologia 41:271-278). Several strategies circumventing this problem have been proposed, some using inhibitors of DPP-IV and others DPP-IV resistant analogues of GLP-1(7-36)amide (Deacon, 1998, Diabetologia 41:271-278; Deacon et al., 1998, Diabetes 47:764-769; Ritzel, 1998, J. Endocrinol. 159:93-102; U.S. Patent No. 5,545,618; Pederson, 1998, Diabetes 47:1253-1258).

Exendins, another group of peptides that lower blood glucose levels have some sequence similarity (53%) to GLP-1[7-36]NH2 (Goke et al., 1993, J. Biol. Chem. 268:19650-55). The exendins are found in the venom of Helodermatidae or beaded lizards (Raufman, 1996, Reg. Peptides 61:1-18). Exendin-3 is present in the venom of *Heloderma horridum,* the Mexican beaded lizard and exendin-4 is present in the venom of *Heloderma suspectum,* the Gila monster. Exendin-4 differs from exendin-3 at just positions two and three. The cDNA encoding the exendin-4 precursor protein, a 47 amino acid peptide fused to the amino terminus of exendin-4 has been cloned and sequenced (Pohl et al., 1998, J. Biol. Chem. 273:9778-9784 and WO98/35033).

Both exendin-3 and exendin-4 stimulate an increase in cellular cAMP production in guinea pig pancreatic acinar cells by interacting with exendin receptors (Raufman, 1996, Reg. Peptides 61:1-18). Exendin-3 causes a biphasic increase in cellular cAMP production, but a monophasic increase in amylase release in pancreatic acinar cells. In contrast, exendin-4 causes a monophasic increase in cAMP production and does not alter amylase release.

Exendin-4 is a strong GLP-1 receptor agonist on isolated rat insulinoma cells (Goke et al., 1993, J. Biol. Chem. 268:19650-55). This is expected as the domain of GLP-1 recognised by DPP-IV is not present in exendin-4 (Goke et al., 1993, J. Biol. Chem. 268:19650-55). Binding of [¹²⁵I]GLP-1 to the nucleus of the solitary tract was inhibited concentration-dependently by unlabelled GLP-1 and [Tyr39]exendin-4 with Ki values of 3.5 and 9.4 nM respectively, and similar values are found in cell lines (Goke et al., 1995, Eur. J. Neurosci. 7:2294-2300 and Goke et al., 1993, J. Biol. Chem. 268:19650-55). Further, exendin-4 given systemically lowers blood glucose levels by 40% in db/db mice (WO99/07404). Recently, Grieg et al. (1999, Diabetologia 42:45-50)has shown a long lasting blood glucose lowering effect of once daily intraperitoneal injection of exendin-4 to diabetic *ob/ob* mice).

The use of exendin-3, exendin-4 and exendin agonists has been proposed for the treatment of diabetes mellitus, reducing gastric motility and delaying gastric emptying and the prevention of hyperglycemia (U.S. Patent No. 5,424,286, WO98/0535 and WO99/07404) as well as for the reduction of food intake (WO98/30231).

### OBJECTIVE OF THE INVENTION

There is a need for compounds that lower blood glucose levels in mammals, are stable and effective when administered orally. Therefore, it is an objective of the invention to provide novel compounds that lower blood glucose levels in mammals.

### SUMMARY OF THE INVENTION

The invention is directed to a novel variant of a parent exendin, wherein said parent exendin has an amino acid sequence having at least an 90% homology to exendin-4 and wherein said variant lowers the blood glucose level in a mammal, binds to a GLP-1 receptor and has at least one modification selected from the group consisting of (a) between one and five deletions at positions 34-39, and (b) contains a Lys at position 40 having a lipophilic substituent attached to the epsilon amino group of said lysine.

Furthermore, the invention is directed to a peptide conjugate comprising a peptide X that lowers the blood glucose level in a mammal and binds to a GLP-1 receptor, wherein X is said variant, an exendin having at least 90% homology to exendin-4, or GLP-1 (7-36) or GLP-1 (7-37) having at least one modification selected from the group consisting of: (i) substitution of D-alanine, serine, glycine or alpha-amino isobutyric acid for alanine at position 8 and (ii) a lipophilic substituent and Z, a peptide sequence, of 4-20 amino acid units covalently bound to said variant, wherein each amino acid unit in said peptide sequence, Z is selected from the group consisting of Ala, Leu, Ser, Thr, Tyr, Asn, Gln, Asp, Glu, Lys, Arg, His, Met, Orn, and amino acid units of the general formula I

-NH-C(R¹)(R²)-C(=O)- (I)

wherein R¹ and R² are selected from the group consisting of hydrogen, C₁₋₆-alkyl, phenyl, and phenyl-methyl, wherein C₁₋₆-alkyl is optionally substituted with from one to three substituents selected from halogen, hydroxy, amino, cyano, nitro, sulfono, and carboxy, and phenyl and phenyl-methyl is optionally substituted with from one to three substituents selected from C₁₋₆-alkyl, C₂₋₆-alkenyl, halogen, hydroxy, amino, cyano, nitro, sulfono, and carboxy, or R¹ and R² together with the carbon atom to which they are bound form a cyclopentyl, cyclohexyl, or cycloheptyl ring, e.g. 2,4-diaminobutanoic acid and 2,3-diaminopropanoic acid; and wherein the ratio between the minimum effective oral dose of said peptide conjugate and the minimum effective oral dose of the peptide, X is at least 1:5.

The present invention also relates to a composition, e.g., a pharmaceutical composition, comprising said exendin variant or said peptide conjugate and a physiologically acceptable carrier, to said exendin variant of said peptide conjugate for use in therapy, a method of treating a disorder and to the use of said exendin variant or said peptide conjugate for the manufacture of a pharmaceutical composition for use in therapy. Specifically, the invention is directed to a method for stimulating insulin release in a mammal comprising administering an effective insulinotropic amount of the exendin variant or peptide conjugate of the present invention, a method of lowering blood glucose level in a mammal comprising administering an amount of the exendin variant or peptide conjugate of the present invention effective to lower blood glucose level in said mammal, a method of reducing gastric motility in a mammal in an amount of the exendin variant or peptide conjugate of the present invention effective to reduce gastric motility, a method of delaying gastric emptying in a mammal in an amount of the exendin variant or peptide conjugate of the present invention effective to delay gastric emptying, a method of inhibiting food uptake in a mammal in an amount of the exendin variant or peptide conjugate of the present invention effective to inhibit food uptake and a method of lowering plasma lipid level in a mammal comprising administering an amount of the exendin variant or peptide conjugate of the present invention effective to lower plasma lipid level in said mammal.

Specifically, the exendin variant or peptide conjugate of the present invention may be used in treatment of diabetes type 1 or type 2, obesity, eating disorders, bone disease, hyperglycemia, metabolic disorders, bone disease, gastric disease and insulin resistance syndrome.

The present invention also relates to methods for the preparation of said exendin variant or said peptide conjugate, by means of recombinant DNA technology comprising the steps of (a) introducing a nucleic acid sequence encoding said variant or conjugate into a host cell and (b) culturing said host cell and (c) isolating said variant or conjugate from the culture or (a) culturing a recombinant host cell comprising a nucleic acid sequence encoding said variant or conjugate under conditions permitting the production of said variant or conjugate and (b) isolating said variant or conjugate from the culture.

The method also relates to methods for the preparation of said peptide conjugate in which peptide X is obtained via recombinant DNA methods by isolating said peptide. X is then conjugated to Z which is attached to a solid support or has been prepared by solid phase synthetic methods. Furthermore, the invention relates to the preparation of the peptide conjugate of the present invention by peptide synthetic methods. Furthermore, the invention relates to the preparation of the peptide conjugate of the present invention by peptide synthetic methods.

Furthermore, the invention relates to the use of a peptide sequence (Z) for the preparation of said peptide conjugate.

### BRIEF DESCRIPTION OF THE FIGURES

Figure 1 shows the effect of ZS42-0009 (des Pro³⁶-exendin-4-NH₂) on blood glucose levels of mice.

Figure 2 shows the effect of ZS42-0010 (des Pro³⁶-exendin-4-Lys₆-NH₂ on the blood glucose levels of mice.

Figure 3 shows the effect of ZS42-0019 (Gly⁸ Lys³⁷(palmitoyl)-GLP1-(7-36)(Human)- Lys₇-NH₂ on the blood glucose levels of mice.

### DETAILED DESCRIPTION OF THE INVENTION

### Exendin Variants

The exendin variant of the present invention is a variant of a parent exendin peptide having at least about 90% homology and most preferably at least about 95% to exendin-4, which have exendin activity, e.g., lowers the blood glucose level in a mammal and binds to a GLP-1 receptor. In a preferred embodiment, the parent exendin peptide has an amino acid sequence which differs by five amino acids, preferably by four amino acids, more preferably by three amino acids, even more preferably by two amino acids from the amino acid sequence of exendin-4.

The homology referred to above of the parent exendin is determined as the degree of identity between two protein sequences indicating a derivation of the first sequence from the second. The homology may suitably be determined by means of computer programs known in the art such as GAP proided in the GCG program package (Program Manual for the Wisconsin Package, Version 8, August 1994, Genetics Computer Group, 575 Science Drive, Madison, Wisconsin, USA 53711) (Needleman, S.B. and Wunsch, C.D., (1970), J. Mol. Biol. 48:443-453). The following settings for polypeptide sequence comparison may be used: GAP creation penalty of 3.0 and GAP extension penalty of 0.1.

In one embodiment, the exendin variant comprises between one and five deletions at positions 34-39.

In another embodiment, the variant comprises an additional residue at position 40, a lysine residue which comprises a lipophilic substituent bound to the epsilon amino group of lysine via an amide bond. The lipophilic substituent may be the acyl group of a straight-chain or branched fatty acid or a straight-chain or branched alkane α,ω-dicarboxylic acid . The acyl group may have the formula CH₃(CH₂)ₙCO-, wherein n is an integer from 4-38 and preferably from 4-24. In a specific embodiment, the acyl group is selected from the group consisting of CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO-, and CH₃(CH₂)₂₂CO-. The acyl group may have the formula HOOC(CH2)mCO-, wherein n is an integer from 4-38 and preferably from 4-24. In a specific embodiment, the acyl group is selected from the group consisting of HOOC(CH₂)₁₄CO-, HOOC(CH₂)₁₆CO-, HOOC(CH₂)₁₈CO-, HOOC(CH₂)₂₀CO- and HOOC(CH₂)₂₂CO-. In a more specific embodiment, the lipophilic substituent is selected from the group consisting of tetradecanoyl, ω-carboxynonadecanoyl, 7-deoxycholoyl, choloyl, palmitoyl and lithocholyl. In a most specific embodiment, the lipophilic substituent is palmitoyl.

Alternatively, the liphophilic substituent may have an NH group. Specific embodiments include but are not limited to the formulae CH₃(CH₂)ₐ((CH₂)_{b}COOH)CHNHCO(CH₂)₂CO-, in which a and b are integers and a+b is an integer of from 8 to 33, preferably from 12 to 28; CH₃(CH₂)_{c}CONCH(COOH) (CH₂)₂CO-, wherein c is an integer of from 10 to 24; CH₃(CH₂)_{d} CONHCH(CH₂)₂ (COOH)CO-, wherein d is an integer of from 8 to 24; COOH(CH₂)ₑCO-, wherein e is an integer of from 8 to 24; -NHCH(COOH)(CH₂)₄NHCO(CH₂)_{f}CH₃, wherein f is an integer of from 8 to 18; -NHCH(COOH)(CH₂)₄NHCOCH(CH₂)₂COOH)NHCO(CH₂)_{g}CH₃, wherein g is an integer of from 10 to 16; -NHCH(COOH)(CH₂)₄NHCO(CH₂)₂ CH(COOH)NHCO(CH₂)ₕCH₃, wherein h is an integer of 0 or from 1 to 22 and preferably from 10 to 16.

The variant may be in a most specific embodiment selected from the group consisting of des Ser³⁹-exendin-4-NH₂, des Pro³⁶-exendin-4-NH₂, des Ala³⁵-exendin-4-NH₂ and des Gly³⁴-exendin-4-NH₂, des Ser³⁹-(Lys⁴⁰ (palmitoyl))exendin-4-NH₂, des Gly³⁴-(Lys⁴⁰ (palmitoyl))exendin-4-NH₂, des Ala³⁵-(Lys⁴⁰ (palmitoyl))exendin-4-NH₂ and des Pro³⁶-(Lys⁴⁰ (palmitoyl))exendin-4-NH₂.

### Peptide Conjugates

The peptide sequence X used in the peptide conjugate of the present invention lowers the blood glucose level of a mammal as determined by assays known in the art for a particular peptide. Examples of such an assay are described herein.

In the present context, the peptide sequence X may be the exendin variant or parent exendin described above. Alternatively, X may be GLP-1 (7-36) or GLP-1 (7-37) having at least one modification selected from the group consisting of a substitution of D-alanine, serine, glycine or alpha-amino isobutyric acid for alanine at position 8 or any of the lipophilic substituents described above. In a specific embodiment, the lipophilic substituent is attached to the epsilon amino group of a lysine residue. In a more specific embodiment, the lipophilic substituent is attached to the epsilon amino group of the lysine residue(s) at position 26, 34 and/or 37. In a most specific embodiment, X is Gly⁸Lys³⁷N-palmitoyl-GLP-1(7-36), Gly⁸ -GLP-1 (7-36), Gly⁸Lys³⁴N-palmitoyl-GLP-1(7-36) or Gly⁸Lys²⁶N-palmitoyl-GLP-1(7-36).

Z is typically a peptide sequence of 4-20 amino acid residues, e.g., in the range of 4-15, more preferably in the range of 4-10 in particular in the range of 4-7 amino acid residues, e.g., of 4, 5, 6 or 7 amino acid residues. Each of the amino acid residues in the peptide sequence Z are independently selected from Ala, Leu, Ser, Thr, Tyr, Asn, Gln, Asp, Glu, Lys, Arg, His, Met, Orn, and amino acids of the formula I as defined herein such as diaminobutanoic acid or diaminopropanoic acid. Preferably, the amino acid residues are selected from Ser, Thr, Tyr, Asn, Gln, Asp, Glu, Lys, Arg, His, Orn, and Met, more preferably from Glu, Lys, and Met, especially Lys. The above-mentioned amino acids may have either D- or L-configuration, but preferably the above-mentioned amino acids have an L-configuration.

Thus, illustrative examples of the peptide sequence Z are: 
Lys-Lys-Lys-Lys, Xaa-Lys-Lys-Lys, Lys-Xaa-Lys-Lys, Lys-Lys-Xaa-Lys, Lys-
Lys-Lys-Xaa, Xaa-Xaa-Lys-Lys, Xaa-Lys-Xaa-Lys, Xaa-Lys-Lys-Xaa, Lys-Xaa-
Xaa-Lys, Lys-Xaa-Lys-Xaa, Lys-Lys-Xaa-Xaa, Xaa-Xaa-Xaa-Lys, Xaa-Xaa-Lys-
Xaa, Xaa-Lys-Xaa-Xaa, Lys-Xaa-Xaa-Xaa, Xaa-Xaa-Xaa-Xaa, Lys-Lys-Lys-Lys-
Lys, Xaa-Lys-Lys-Lys-Lys, Lys-Xaa-Lys-Lys-Lys, Lys-Lys-Xaa-Lys-Lys, Lys-
Lys-Lys-Xaa-Lys, Lys-Lys-Lys-Lys-Xaa, Xaa-Xaa-Lys-Lys-Lys, Xaa-Lys-Xaa-
Lys-Lys, Xaa-Lys-Lys-Xaa-Lys, Xaa-Lys-Lys-Lys-Xaa, Lys-Xaa-Xaa-Lys-Lys,
Lys-Xaa-Lys-Xaa-Lys, Lys-Xaa-Lys-Lys-Xaa, Lys-Lys-Xaa-Xaa-Lys, Lys-Lys-
Xaa-Lys-Xaa, Lys-Lys-Lys-Xaa-Xaa, Lys-Lys-Xaa-Xaa-Xaa, Lys-Xaa-Lys-Xaa-
Xaa, Lys-Xaa-Xaa-Lys-Xaa, Lys-Xaa-Xaa-Xaa-Lys, Xaa-Lys-Lys-Xaa-Xaa, Xaa-
Lys-Xaa-Xaa-Lys, Xaa-Xaa-Lys-Lys-Xaa, Xaa-Xaa-Lys-Xaa-Lys, Xaa-Xaa-Xaa-
Lys-Lys, Lys-Xaa-Xaa-Xaa-Xaa, Xaa-Lys-Xaa-Xaa-Xaa, Xaa-Xaa-Lys-Xaa-Xaa,
Xaa-Xaa-Xaa-Lys-Xaa, Xaa-Xaa-Xaa-Xaa-Lys, Xaa-Xaa-Xaa-Xaa-Xaa, Lys-Lys-
Lys-Lys-Lys-Lys, Xaa-Lys-Lys-Lys-Lys-Lys, Lys-Xaa-Lys-Lys-Lys-Lys, Lys-
Lys-Xaa-Lys-Lys-Lys, Lys-Lys-Lys-Xaa-Lys-Lys, Lys-Lys-Lys-Lys-Xaa-Lys,
Lys-Lys-Lys-Lys-Lys-Xaa, Xaa-Xaa-Lys-Lys-Lys-Lys, Xaa-Lys-Xaa-Lys-Lys-
Lys, Xaa-Lys-Lys-Xaa-Lys-Lys, Xaa-Lys-Lys-Lys-Xaa-Lys, Xaa-Lys-Lys-Lys-
Lys-Xaa, Lys-Xaa-Xaa-Lys-Lys-Lys, Lys-Xaa-Lys-Xaa-Lys-Lys, Lys-Xaa-Lys-
Lys-Xaa-Lys, Lys-Xaa-Lys-Lys-Lys-Xaa, Lys-Lys-Xaa-Xaa-Lys-Lys, Lys-Lys-
Xaa-Lys-Xaa-Lys, Lys-Lys-Xaa-Lys-Lys-Xaa, Lys-Lys-Lys-Xaa-Xaa-Lys, Lys-
Lys-Lys-Xaa-Lys-Xaa, Lys-Lys-Lys-Lys-Xaa-Xaa, Xaa-Xaa-Xaa-Lys-Lys-Lys,
Xaa-Xaa-Lys-Xaa-Lys-Lys, Xaa-Xaa-Lys-Lys-Xaa-Lys, Xaa-Xaa-Lys-Lys-Lys-
Xaa, Xaa-Lys-Xaa-Xaa-Lys-Lys, Xaa-Lys-Xaa-Lys-Xaa-Lys, Xaa-Lys-Xaa-Lys-
Lys-Xaa, Xaa-Lys-Lys-Xaa-Xaa-Lys, Xaa-Lys-Lys-Xaa-Lys-Xaa, Xaa-Lys-Lys-
 
Lys-Xaa-Xaa, Lys-Lys-Lys-Xaa-Xaa-Xaa, Lys-Lys-Xaa-Lys-Xaa-Xaa, Lys-Lys-
Xaa-Xaa-Lys-Xaa, Lys-Lys-Xaa-Xaa-Xaa-Lys, Lys-Xaa-Lys-Lys-Xaa-Xaa, Lys-
Xaa-Lys-Xaa-Lys-Xaa, Lys-Xaa-Lys-Xaa-Xaa-Lys, Lys-Xaa-Xaa-Lys-Lys-Xaa,
Lys-Xaa-Xaa-Lys-Xaa-Lys, Lys-Xaa-Xaa-Xaa-Lys-Lys, Lys-Lys-Xaa-Xaa-Xaa-
Xaa, Lys-Xaa-Lys-Xaa-Xaa-Xaa, Lys-Xaa-Xaa-Lys-Xaa-Xaa-Lys, Lys-Xaa-Xaa-
Xaa-Lys-Xaa-Lys, Lys-Xaa-Xaa-Xaa-Xaa-Lys-Lys, Xaa-Lys-Lys-Xaa-Xaa-Xaa,
Xaa-Lys-Xaa-Lys-Xaa-Xaa, Xaa-Lys-Xaa-Xaa-Lys-Xaa, Xaa-Lys-Xaa-Xaa-Xaa-
Lys, Xaa-Xaa-Lys-Lys-Xaa-Xaa, Xaa-Xaa-Lys-Xaa-Lys-Xaa, Xaa-Xaa-Lys-Xaa-
Xaa-Lys, Xaa-Xaa-Xaa-Lys-Lys-Xaa, Xaa-Xaa-Xaa-Lys-Xaa-Lys, Xaa-Xaa-Xaa-
Xaa-Lys-Lys, Lys-Xaa-Xaa-Xaa-Xaa-Xaa, Xaa-Lys-Xaa-Xaa-Xaa-Xaa, Xaa-Xaa-
Lys-Xaa-Xaa-Xaa, Xaa-Xaa-Xaa-Lys-Xaa-Xaa, Xaa-Xaa-Xaa-Xaa-Lys-Xaa, Xaa-
Xaa-Xaa-Xaa-Xaa-Lys, Xaa-Xaa-Xaa-Xaa-Xaa-Xaa, Lys-Lys-Lys-Lys-Lys-Lys-
Lys, Xaa-Lys-Lys-Lys-Lys-Lys-Lys, Lys-Xaa-Lys-Lys-Lys-Lys-Lys, Lys-Lys-
Xaa-Lys-Lys-Lys-Lys, Lys-Lys-Lys-Xaa-Lys-Lys-Lys, Lys-Lys-Lys-Lys-Xaa-
Lys-Lys, Lys-Lys-Lys-Lys-Lys-Xaa-Lys, Lys-Lys-Lys-Lys-Lys-Lys-Xaa, Xaa-
Xaa-Lys-Lys-Lys-Lys-Lys, Xaa-Lys-Xaa-Lys-Lys-Lys-Lys, Xaa-Lys-Lys-Xaa-
Lys-Lys-Lys, Xaa-Lys-Lys-Lys-Xaa-Lys-Lys, Xaa-Lys-Lys-Lys-Lys-Xaa-Lys,
Lys-Xaa-Xaa-Lys-Lys-Lys-Lys, Lys-Xaa-Lys-Xaa-Lys-Lys-Lys, Lys-Xaa-Lys-
Lys-Xaa-Lys-Lys, Lys-Xaa-Lys-Lys-Lys-Xaa-Lys, Lys-Lys-Xaa-Xaa-Lys-Lys-
Lys, Lys-Lys-Xaa-Lys-Xaa-Lys-Lys, Lys-Lys-Xaa-Lys-Lys-Xaa-Lys, Lys-Lys-
Lys-Xaa-Xaa-Lys-Lys, Lys-Lys-Lys-Xaa-Lys-Xaa-Lys, Lys-Lys-Lys-Lys-Xaa-
Xaa-Lys, Xaa-Xaa-Xaa-Lys-Lys-Lys-Lys, Xaa-Xaa-Lys-Xaa-Lys-Lys-Lys, Xaa-
Xaa-Lys-Lys-Xaa-Lys-Lys, Xaa-Xaa-Lys-Lys-Lys-Xaa-Lys, Xaa-Lys-Xaa-Xaa-
Lys-Lys-Lys, Xaa-Lys-Xaa-Lys-Xaa-Lys-Lys, Xaa-Lys-Xaa-Lys-Lys-Xaa-Lys,
Xaa-Lys-Lys-Xaa-Xaa-Lys-Lys, Xaa-Lys-Lys-Xaa-Lys-Xaa-Lys, Xaa-Lys-Lys-
Lys-Xaa-Lys-Xaa, Xaa-Lys-Lys-Xaa-Lys-Lys-Xaa, Xaa-Lys-Xaa-Lys- Lys-Lys-
Xaa, Xaa-Lys-Lys-Lys-Xaa-Xaa-Lys, Lys-Xaa-Lys-Lys-Lys-Xaa-Xaa, Xaa-Lys-
Lys-Lys-Lys-Xaa-Xaa, Xaa-Lys-Lys-Lys-Xaa-Lys-Xaa, Xaa-Lys-Lys-Lys-Xaa-
Xaa-Lys, Lys-Lys-Lys-Lys-Xaa-Xaa-Xaa, Lys-Lys-Lys-Xaa-Xaa-Xaa-Lys, Lys-
 
Lys-Lys-Xaa-Lys-Xaa-Xaa, Lys-Lys-Xaa-Lys-Lys-Xaa-Xaa, Lys-Lys-Xaa-Xaa-
Lys-Xaa-Lys, Lys-Lys-Xaa-Xaa-Xaa-Lys-Lys, Lys-Lys-Xaa-Lys-Lys-Xaa-Xaa,
Lys-Xaa-Lys-Lys-Xaa-Xaa-Lys, Lys-Xaa-Lys-Xaa-Lys-Xaa-Lys, Lys-Xaa-Lys-
Xaa-Xaa-Lys-Lys, Lys-Xaa-Xaa-Lys-Lys-Xaa-Lys, Lys-Xaa-Xaa-Lys-Xaa-Lys-
Lys, Lys-Xaa-Xaa-Xaa-Lys-Lys-Lys, Lys-Lys-Xaa-Xaa-Xaa-Xaa-Lys, Lys-Xaa-
Lys-Xaa-Xaa-Xaa-Lys, Lys-Xaa-Xaa-Lys-Xaa-Xaa-Lys, Lys-Xaa-Xaa-Xaa-Lys-
Xaa-Lys, Lys-Xaa-Xaa-Xaa-Xaa-Lys-Lys, Xaa-Lys-Lys-Xaa-Xaa-Xaa-Lys, Xaa-
Lys-Xaa-Lys-Xaa-Xaa-Lys, Xaa-Lys-Xaa-Xaa-Lys-Xaa-Lys, Xaa-Lys-Xaa-Xaa-
Xaa-Lys-Lys, Xaa-Xaa-Lys-Lys-Xaa-Xaa-Lys, Xaa-Xaa-Lys-Xaa-Lys-Xaa-Lys,
Xaa-Xaa-Lys-Xaa-Xaa-Lys-Lys, Xaa-Xaa-Xaa-Lys-Lys-Xaa-Lys, Xaa-Xaa-Xaa-
Lys-Xaa-Lys-Lys, Xaa-Xaa-Xaa-Xaa-Lys-Lys-Lys, Lys-Xaa-Xaa-Xaa-Xaa-Xaa-
Lys, Xaa-Lys-Xaa-Xaa-Xaa-Xaa-Lys, Xaa-Xaa-Lys-Xaa-Xaa-Xaa-Lys, Xaa-Xaa-
Xaa-Lys-Xaa-Xaa-Lys, Xaa-Xaa-Xaa-Xaa-Lys-Xaa-Lys, Xaa-Xaa-Xaa-Xaa-Xaa-
Lys-Lys, Xaa-Xaa-Xaa-Xaa-Xaa-Xaa-Lys, Lys-Xaa-Xaa-Xaa-Xaa-Xaa-Xaa, Xaa-
Xaa-Xaa-Xaa-Xaa-Lys-Xaa, Xaa-Lys-Xaa-Xaa-Xaa-Xaa-Xaa, Xaa-Xaa-Lys-Xaa-
Xaa-Xaa, Xaa-Xaa-Xaa-Xaa-Lys-Xaa, Xaa-Xaa-Xaa-Xaa-Xaa-Xaa-Xaa,
 wherein each Xaa is independently selected from the group consisting of Ala, Leu, Ser, Thr, Tyr, Asn, Gln, Asp, Glu, Arg, His, Met, Orn, and amino acids of the formula I as defined herein, e.g., Dbu or Dpr.

As indicated above, the amino acid residues of Z may of course all be different or all be identical. However, in interesting embodiments of the present invention, the amino acid residues in Z are selected from two or three different amino acids, or are identical amino acids. Examples of suitable peptide sequences, wherein the amino acid residues in Z are identical are e.g., (Lys)ₙ, wherein n is an integer in the range from 4 to 15, preferably in the range from 4 to 10, such as in the range from 4 to 8, e.g., in the range from about 4 to 7, e.g., Lys₄, Lys₅ Lys₆ , Lys₇. Examples of suitable peptide sequences, wherein the amino acid residues in Z are selected from about two different amino acids are e.g., (Lys-Xaa)ₘ or (Xaa-Lys)ₘ, wherein m is an integer in the range from about 2 to 7, preferably in the range from 2 to 5, such as in the range from 2 to 4, e.g., 3, and Xaa is independently selected from the group consisting of Ser, Thr, Tyr, Asn, Gln, Asp, Glu, Arg, His, Orn, 2,4-diaminobutanoic acid, 2,3-diaminopropanoic acid and Met. More preferably such peptide sequences are e.g., (Lys-Xaa)₃ or (Xaa-Lys)₃, wherein Xaa is as defined above, such as (Lys-Glu)₃ or (Clu-Lys)₃. Other examples of suitable peptide sequences, wherein the amino acid residues in Z are selected from about two amino acid residues are e.g., Lysₚ-Xaa_{q} or Xaa_{P}-Lys_{q}, wherein p and q are integers in the range from 1 to 14, with the proviso that p+q is in the range from 4 to 15, preferably in the range from 4 to 10, such as in the range from 4 to 8, e.g., in the range from 4 to 6, e.g., 4, 5 or 6, and Xaa is independently selected from the group consisting of Ser, Thr, Tyr, Asn, Gln, Asp, Glu, Arg, His and Met. More preferably such peptide sequences are e.g., Lys₃-Xaa₃ or Xaa₃-Lys₃, wherein Xaa is as defined above, such as Lys₃-Glu₃ or Glu₃-Lys₃.

Examples of suitable peptide sequences, wherein the amino acid residues in Z are selected from three different amino acids are e.g., Xaa¹-(Lys)ₓ-(Xaa²)_{y}, Xaa¹-(Xaa²)ₓ-(Lys)_{y}, (Lys)ₓ-(Xaa²)_{y}-Xaa¹, (Xaa¹)ₓ-(Lys)_{y}-Xaa², (Lys)ₓ-Xaa¹-(Xaa²)_{y}, (Xaa¹)ₓ-Xaa²-(Lys)_{y}, Xaa¹-Lys-Xaa²-Lys, Xaa¹-Lys-Xaa²-Lys-Xaa², Xaa¹-Lys-Xaa²-Lys-Xaa²-Lys, Xaa¹-Xaa²-Lys-Xaa², Xaa¹-Xaa²-Lys-Xaa²-Lys, Xaa¹-Xaa¹-Lys-Xaa²-Lys-Xaa², Lys-Xaa²-Lys-Xaa¹, Lys-Xaa²-Lys-Xaa²-Xaa¹, Lys-Xaa²-Lys-Xaa²-Lys-Xaa¹, Xaa²-Lys-Xaa²-Xaa¹, Xaa²-Lys-Xaa²-Lys-Xaa¹, Xaa²-Lys-Xaa¹-Lys-Xaa²-Xaa¹, etc., wherein x and y are integers in the range from about 1 to 5 with the proviso that x+y is at the most 6, and Xaa¹ and Xaa² is independently selected from about the group consisting of Ala, Leu, Ser, Thr, Tyr, Asn, Gln, Asp, Glu, Arg, His, Met, Orn, 2,3-diaminopropanoic acid, 2,4-diaminobutanoic acid and amino acids of the formula I as defined herein.

The peptide sequence Z may be bound to the C-terminal or the N-terminal of the peptide sequence, X, or two peptide sequences may be bound individually to both the C- and N-terminal of X. In case the native peptide X possesses a free C-terminal carboxylic acid, the peptide sequence Z may be attached to either the C-terminal of the peptide X or to the N-terminal of the peptide X, or the C- and N-terminal of X may both be bound to each individual peptide sequence Z. Alternatively, Z may be bound to the nitrogen atom on the side chain of lysine, histidine or arginine or a carbonyl function on the side chain of glutamic acid or aspartic acid anywhere within the peptide sequence X. In one embodiment, Z may be attached to X within the sequence and to the N- and/or C-terminal of X. Whether the sequence should be attached to the peptide sequence X at its C-terminal, at its N-terminal, or both, or within the peptide sequence X depends on the specific peptide X and can be easily determined by the person skilled in the art.

It should be understood that the peptide conjugates of the invention might also be in the form of a salt thereof. Salts include pharmaceutically acceptable salts, such as acid addition salts and basic salts. Examples of acid addition salts are hydrochloride salts, sodium salts, calcium salts, potassium salts, etc. Examples of basic salts are salts where the cation is selected from alkali metals, such as sodium and potassium, alkaline earth metals, such as calcium, and ammonium ions ⁺N (R³) ₃(R⁴)_{,} where R³ and R⁴ independently designates optionally substituted C₁₋₆-alkyl, optionally substituted C₂₋₆-alkenyl, optionally substituted aryl, or optionally substituted heteroaryl. Other examples of pharmaceutically acceptable salts are; e.g., those described in "Remington's Pharmaceutical Sciences" 17. Ed. Alfonso R. Gennaro (Ed.), Mark Publishing Company, Easton, PA, U.S.A., 1985 and more recent editions, and in Encyclopedia of Pharmaceutical Technology.

As explained above, the peptide sequence Z is the part of the peptide conjugate responsible for introducing of a certain structure into the molecule so that the minimum effective dose is lowered at least five fold. Preferably the minimum effective dose is lowered at least ten fold, more preferably 25 fold, even more preferably 40 fold, and most preferably 50 fold. Therefore, the present invention also relates to the use of a peptide sequence (Z) as defined above for the preparation of a said peptide conjugate as defined above.

In a most specific embodiment, the conjugates are selected from the group consisting of Gly⁸-GLP-1-(7-36)(Human)- NH₂, Gly⁸-GLP-1-(7-36)(Human)-Lys₆-NH₂, Gly⁸Lys³⁷(palmitoyl) -GLP-1-(7-36)(Human)-Lys₇-NH₂, Gly⁸Lys³⁴(palmitoyl) -GLP-1-(7-36)(Human)-Lys₆-NH₂, des Ser³⁹-exendin-4-Lys₆-NH₂, exendin-4-Lys₆-NH2, des Pro³⁶-exendin-4- Lys₆-NH₂, des Ala³⁵-exendin-4-Lys₆-NH₂, des Gly³⁴-exendin-4-Lys₆-NH₂, des Ser³⁹-(Lys⁴⁰ (palmitoyl))exendin-4-Lys₇-NH₂, des Gly³⁴-(Lys⁴⁰ (palmitoyl))exendin-4-Lys₇-NH₂, des Ala³⁵-(Lys⁴⁰ (palmitoyl))exendin-4-Lys₇-NH₂, des Pro³⁶-(Lys⁴⁰ (palmitoyl))exendin-4-Lys₇-NH₂ and Lys⁴⁰ (palmitoyl)exendin-4-Lys₇-NH₂.

### Compositions

The invention also concerns a composition comprising the exendin variant or the peptide conjugate of the present invention in combination with a physiologically acceptable carrier. Such compositions may be in a form adapted to oral, subcutaneous, parenteral (intravenous, intraperitoneal), intramuscular, rectal, epidural, intratracheal, intranasal, dermal, vaginal, buccal, ocularly, direct brain or pulmonary administration, preferably in a form adapted to oral administration, and such compositions may be prepared in a manner well-known to the person skilled in the art, e.g., as generally described in "Remington's Pharmaceutical Sciences", 17. Ed. Alfonso R. Gennaro (Ed.), Mark Publishing Company, Easton, PA, U.S.A., 1985 and more recent editions and in the monographs in the "Drugs and the Pharmaceutical Sciences" series, Marcel Dekker. The compositions may appear in conventional forms, for example, capsules, tablets, aerosols, solutions, suspensions or topical applications. In a preferred embodiment, the compositions of the present invention are administered orally.

The pharmaceutical carrier or diluent employed may be a conventional solid or liquid carrier. Examples of solid carriers are lactose, terra alba, sucrose, cyclodextrin, talc, gelatin, agar, pectin, acacia, magnesium stearate, stearic acid ro lower alkyl ethers of cellulose. Examples of liquid carriers are syrup, peanut oil, olive oil, phospholipids, fatty acids, fatty acid amines, polyoxyethylene and water.

Similarly, the carrier or diluent may include any sustained release material known in the art, such as glyceryl monostearate or glyceryl distearate, alone or mixed with a wax.

If a solid carrier is used for oral administration, the preparation may be tabletted, placed in a hard gelatin capsule in powder or pellet form or it can be in the form of a troche or lozenge. The amount of solid carrier will vary widely but will usually be from about about 25 mg to about 1 g.

A typical tablet which may be prepared by conventional tabletting techniques may contain:

Core: active compound (as free compound or salt thereof) 100 mg; colloidal silicon dioxide (Aerosil) 1.5 mg; cellulose, microcryst. (Avicel) 70 mg; modified cellulose gum (Ac-Di-Sol) 7.5 mg; magnesium stearate.

Coating: HPMC approx. 9 mg; *Mywacett 9-40T approx. 0.9 mg; ∗acylated monoglyceride used as plasticizer for film coating.

If a liquid carrier is used, the preparation may be in the form of a syrup, emulsion, soft gelatin capsule or sterile injectable liquid such as an aqueous or non-aqueous liquid suspension or solution.

For nasal administration, the preparation may contain a conjugate of the present invention dissolved or suspended in a liquid carrier, in particular, an aqueous carrier, for aerosol application. The carrier may contain additives such as solubilizing agents, e.g., propylene glycol, surfactants such as bile acid salts or polyoxyethylene higher alcohol ethers, absorption enhancers such as lecithin (phosphatidylcholine) or cyclodextrin, or preservatives such as parabines.

The composition may also be in a form suited for local or systemic injection or infusion and may, as such, be formulated with sterile water or an isotonic saline or glucose solution. The compositions may be sterilized by conventional sterilization techniques which are well known in the art. The resulting aqueous solutions may be packaged for use or filtered under aseptic conditions and lyophilized, the lyophilized preparation being combined with the sterile aqueous solution prior to administration. The composition may contain pharmaceutically acceptable auxiliary substances as required to approximate physiological conditions, such as buffering agents, tonicity adjusting agents and the like, for instance sodium acetate, sodium lactate, sodium chloride, potassium chloride, calcium chloride, etc.

The invention also concerns the exendin variants and peptide conjugates as defined above for use in therapy, and the use of the exendin variants and peptide conjugates as defined above for the manufacture of a pharmaceutical composition for use in therapy, e.g., in the treatment of diabetes type 1 or type 2, obesity, eating disorders and insulin resistance syndrome.

In specific embodiments, the variants or conjugates of the invention may be used to stimulate insulin release, lower blood glucose level, reduce gastric motility, delay gastric emptying, inhibit food uptake or lower the plasma lipid level in a mammal.

### Preparation of Variants and Conjugates

The variants and the peptide conjugates of the invention may be prepared by methods known per se in the art. Thus, the variants and the peptide sequences X and Z may be prepared by standard peptide-preparation techniques such as solution synthesis or Merrifield-type solid phase synthesis.

In one possible synthesis strategy, the peptide conjugates of the invention may be prepared by solid phase synthesis by first constructing the peptide sequence Z using well-known standard protection, coupling and deprotection procedures, thereafter sequentially coupling the peptide sequence X on Z in a manner similar to the construction of Z, and finally cleaving off the entire peptide conjugate from the carrier. This strategy yields a peptide conjugate, wherein the peptide sequence Z is covalently bound to the peptide X at the C-terminal carbonyl function of X. If the desired peptide conjugate, however, is a peptide conjugate, wherein two stabilising sequences Z are covalently and independently bound to both the C- and the N-terminal of the peptide X, the above strategy is also applicable but, as will be understood by the person skilled in the art, before cleaving the off the C-terminal bound peptide conjugate from the solid support, it is necessary to sequentially couple the second peptide sequence Z to the N-terminal of X in a manner similar to the procedure described above. This strategy may also be used to attach Z to the carbonyl function on the side chain of Glu or Asp.

A possible strategy for the preparation of peptide conjugates, wherein the peptide sequence Z is covalently bound to the N-terminal nitrogen atom or covalently bound to the nitrogen atom on the side chain of Lys, Arg or His of X is analogous with the method described above, i.e. said peptide conjugates may be prepared by solid phase synthesis by first constructing the peptide sequence X using well-known standard protection, coupling and deprotection procedures, thereafter sequentially coupling the peptide sequence Z on X in a manner similar to the construction of X, and finally cleaving off the entire peptide conjugate from the carrier.

Another possible strategy is to prepare one or both of the two sequences X and Z (or parts thereof) separately by solution synthesis, solid phase synthesis, recombinant techniques, or enzymatic synthesis, followed by coupling of the two sequences by well-known segment condensation procedures, either in solution or using solid phase techniques or a combination thereof. In one embodiment, X may be prepared by recombinant DNA methods and Z may be prepared by solid phase synthesis. The conjugation of X and Z may be carried out by using chemical ligation. This technique allows for the assembling of totally unprotected peptide segments in a highly specific manner (Liu et al., 1996, J. Am. Chem. Soc. 118:307-312 and Dawson et al., 1996, 226:776). The conjugation can also be performed by protease-catalysed peptide bond formation, which offers a highly specific technique to combine totally unprotected peptide segments via a peptide bond (W. Kullmann, 1987, Enzymatic Peptide Synthesis, CRC Press, Boca Raton, Florida, pp. 41-59).

Side chain derivatization of Lys, Arg, His, Trp, Ser, Thr, Cys, Tyr, Asp and Glu with the peptide sequence, Z can be carried out by traditional convergent peptide synthesis using suitable orthogonal protecting schemes as known in the art, or by using the equally well known general solid phase method with suitable orthogonal removable chain protection.

Furthermore, it is envisaged that a combination of the above-mentioned strategies may be especially applicable where a modified peptide sequence, e.g., from a peptide X comprising isosteric bonds such as reduced peptide bonds, is to be coupled to a peptide sequence Z. In this case, it may be advantageous to prepare the immobilised fragment of Z by successive coupling of amino acids, and then couple a complete peptide sequence X (prepared in solution or fully or partially using solid phase techniques or by means of recombinant techniques) to the fragment.

Examples of suitable solid support materials (SSM) are e.g., functionalised resins such as polystyrene, polyacrylamide, polydimethylacrylamide, polyethyleneglycol, cellulose, polyethylene, polyethyleneglycol grafted on polystyrene, latex, dynabeads, etc.

It should be understood that it may be necessary or desirable that the C-terminal amino acid of the peptide sequence Z or the C-terminal amino acid of the peptide X is attached to the solid support material by means of a common linker such as 2,4-dimethoxy-4'-hydroxy-benzophenone, 4-(4-hydroxy-methyl-3-methoxyphenoxy)-butyric acid, 4-hydroxy-methylbenzoic acid, 4-hydroxymethyl-phenoxyacetic acid, 3-(4-hydroxymethylphenoxy)propionic acid, and p-[(R,S)-a[1-(9H-fluoren-9-yl)methoxyformamido]-2,4-dimethoxybenzyl]-phenoxy-acetic acid.

The peptide conjugates of the invention may be cleaved from the solid support material by means of an acid such as trifluoracetic acid, trifluoromethanesulfonic acid, hydrogen bromide, hydrogen chloride, hydrogen fluoride, etc. optionally in combination with one or more "scavengers" suitable for the purpose, e.g., ethanedithiol, triisopropylsilane, phenol, thioanisole, etc., or the peptide conjugate of the invention may be cleaved from the solid support by means of a base such as ammonia, hydrazine, an alkoxide, such as sodium ethoxide, an hydroxide, such as sodium hydroxide, etc.

Thus, the present invention also relates to a method for the preparation of a pharmacologically active peptide conjugate, wherein Z is covalently bound to X at the C-terminal function of X (X-Z), comprising the steps of:
a) coupling an N-α-protected amino acid in the carboxyl activated form, or an N-α-protected dipeptide in the C-terminal activated form to an immobilised peptide sequence H-Z-SSM, thereby forming an immobilised N-α-protected peptide fragment,
b) removing the N-α-protecting group, thereby forming an immobilised peptide fragment having an unprotected N-terminal end,
c) coupling an additional N-α-protected amino acid in the carboxyl activated form, or an additional N-α-protected dipeptide in the C-terminal activated form to the N-terminal end of the immobilised peptide fragment, and repeating the removal/coupling step procedure in step b) and c) until the desired peptide sequence X is obtained, and then
d) cleaving off the peptide conjugate from the solid support material.

In a further aspect the present invention also relates to a method for the preparation of a pharmacologically active peptide conjugate, wherein Z is covalently bound to the N-terminal nitrogen atom of X (Z-X), comprising the steps of:
a) coupling an N-α-protected amino acid, or an N-α-protected dipeptide to a solid support material (SSM), thereby forming an immobilised N-α-protected amino acid, or an immobilised N-α-protected dipeptide fragment,
b) removing the N-α-protecting group, thereby forming an immobilised amino acid or peptide fragment having an unprotected N-terminal end,
c) coupling an additional N-α-protected amino acid in the carboxyl activated form, or an additional N-α-protected dipeptide in the C-terminal activated form to the N-terminal end of the immobilised amino acid or peptide fragment, and repeating the removal/coupling step procedure in step b) and c) until the desired peptide sequence X is obtained,
d) coupling an additional N-α-protected amino acid in the carboxyl activated form, or an additional N-α-protected dipeptide in the C-terminal activated form to the N-terminal end of the immobilised peptide fragment, and
   repeating the removal/coupling step procedure in step b) and d) until the desired peptide sequence Z is obtained, and then
e) cleaving off the peptide conjugate from the solid support material.

In a still further aspect, the present invention relates to a method for the preparation of the peptide conjugate of the present invention, wherein a first sequence (Z) is covalently bound to X at the C-terminal function of X and a second sequence (Z) is covalently bound to the N-terminal nitrogen atom of X (Z-X-Z), comprising the steps of:
a) coupling an N-α-protected amino acid in the carboxyl activated form, or an N-α-protected dipeptide in the C-terminal activated form to an immobilised peptide sequence H-Z-SSM, thereby forming an immobilised N-α-protected peptide fragment,
b) removing the N-α-protecting group, thereby forming an immobilised peptide fragment having an unprotected N-terminal end,
c) coupling an additional N-α-protected amino acid in the carboxyl activated form, or an additional N-α-protected dipeptide in the C-terminal activated form to the N-terminal end of the immobilised peptide fragment, and repeating the removal/coupling step procedure in step b) and c) until the desired peptide sequence X is obtained, and then
d) coupling an additional N-α-protected amino acid in the carboxyl activated form, or an additional N-α-protected dipeptide in the C-terminal activated form to the N-terminal end of the immobilised peptide fragment, and repeating the removal/coupling step procedure in step b) and d) until the desired peptide sequence Z is obtained, and then
e) cleaving off the peptide conjugate from the solid support material.

The coupling, removal and cleavage steps are performed by methods known to the person skilled in the art taking into consideration the protection strategy and the selected solid phase material. In general, however, it is believed that the Boc (tert.butyloxycarbonyl) as well as the Fmoc (9-fluorenylmethyloxycarbonyl) protection strategies are applicable and that peptide bonds may be formed using the various activation procedures known to the person skilled in the art, e.g., by reacting a C-terminal activated derivative (acid halide, acid anhydride, activated ester e.g., HObt-ester, etc.) of the appropriate amino acid or peptide with the amino group of the relevant amino acid or peptide as known to a person skilled in peptide chemistry.

Furthermore, it may be necessary or desirable to include side-chain protection groups when using amino acid residues carrying functional groups which are reactive under the prevailing conditions. The necessary protection scheme will be known to the person skilled in the art (see e.g., M. Bodanszky and A. Bodanszky, "The Practice of Peptide Synthesis", 2. Ed, Springer-Verlag, 1994, J. Jones, "The Chemical Synthesis of Peptides", Clarendon Press, 1991, and Dryland et al., 1986, J. Chem. Soc., Perkin Trans. 1:125-137).

The peptide conjugates may also be prepared by means of recombinant DNA technology using general methods and principles known to the person skilled in the art. A nucleic acid sequence encoding the conjugate may be prepared synthetically by established standard methods, e.g., the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to the phosphoamidite method, oligonucleotides are synthesized, e.g., in an automatic DNA synthesizer, purified, annealed, ligated and cloned in suitable vectors.

The techniques used to isolate or clone a nucleic acid sequence encoding peptide X or exendin variant are known in the art and include isolation from genomic DNA, preparation from cDNA, or a combination thereof. The cloning of the nucleic acid sequences of the present invention from such genomic DNA can be effected, e.g., by using the well known polymerase chain reaction (PCR) or antibody screening of expression libraries to detect cloned DNA fragments with shared structural features. See, e.g., Innis et al., 1990, A Guide to Methods and Application, Academic Press, New York. Other nucleic acid amplification procedures such as ligase chain reaction (LCR), ligated activated transcription (LAT) and nucleic acid sequence-based amplification (NASBA) may be used. It can then be ligated to a nucleic acid sequence encoding Z.

The nucleic acid sequence encoding the conjugate is then inserted into a recombinant expression vector which may be any vector which may conveniently be subjected to recombinant DNA procedures. The choice of vector will often depend on the host cell into which it is to be introduced. Thus, the vector may be an autonomously replicating vector, i.e., a vector which exists as an extrachromosomal entity, the replication of which is independent of chromosomal replication, e.g., a plasmid. Alternatively, the vector may be one which, when introduced into a host cell, is integrated into the host cell genome and replicated together with the chromosome(s) into which it has been integrated.

In the vector, the nucleic acid sequence encoding the conjugate of the present invention should be operably connected to a suitable promoter sequence. The promoter may be any nucleic acid sequence which shows transcriptional activity in the host cell of choice and may be derived from genes encoding proteins either homologous or heterologous to the host cell. Examples of suitable promoters for directing the transcription of the nucleic acid sequence encoding said conjugate in mammalian cells are the SV 40 promoter (Subramani et al., Mol. Cell Biol. 1, 1981, pp. 854-864), the MT-1 (metallothionein gene) promoter (Palmiter et al., Science 222, 1983, pp. 809-814) or the adenovirus 2 major late promoter, a Rous sarcoma virus (RSV) promoter, cytomegalovirus (CMV) promoter (Boshart et al., 1981, Cell 41:521-530) and a bovine papilloma virus promoter (BPV). A suitable promoter for use in insect cells is the polyhedrin promoter (Vasuvedan et al., FEBS Lett. 311, 1992, pp. 7-11).

Examples of suitable promoters for directing the transcription of the nucleic acid sequence encoding the conjugate, especially in a bacterial host cell, are the promoters obtained from the E. *coli* lac operon, the *Streptomyces coelicolor* agarase gene (dagA), the *Bacillus subtilis* levansucrase gene (sacB), the *Bacillus licheniformis* alpha-amylase gene (amyL), the *Bacillus stearothermophilus* maltogenic amylase gene (amyM), the *Bacillus amyloliquefaciens* alpha amylase gene (amyQ), the *Bacillus licheniformis* penicillinase gene (penP), the *Bacillus subtilis* xylA and xylB genes, and the prokaryotic beta-lactamase gene (Villa-Kamaroff et al., 1978, Proceedings of the National Academy of Sciences USA 75:3727-3731), as well as the tac promoter (DeBoer et al., 1983, Proceedings of the National Academy of Sciences USA 80:21 25). Further promoters are described in "Useful proteins from recombinant bacteria" in Scientific American, 1980, 242:74-94; and in Sambrook et al., 1989, supra.

Examples of suitable promoters for directing the transcription of the nucleic acid sequence encoding the conjugate in a filamentous fungal host cell are promoters obtained from the genes encoding *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (glaA), *Rhizomucor miehei* lipase, *Aspergillus oryzae* alkaline protease, *Aspergillus oryzae* triose phosphate isomerase, Aspergillus nidulans acetamidase, *Fusarium oxysporum* trypsin-like protease (as described in U.S. Patent No. 4,288,627, which is incorporated herein by reference), and hybrids thereof. Particularly preferred promoters for use in filamentous fungal host cells are the TAKA amylase, NA2-tpi (a hybrid of the promoters from the genes encoding *Aspergillus niger* neutral a amylase and *Aspergillus oryzae* triose phosphate isomerase), and glaA promoters.

In a yeast host, useful promoters are obtained from the *Saccharomyces cerevisiae* enolase (ENO-1) gene, the *Saccharomyces cerevisiae* galactokinase gene (GAL1), the *Saccharomyces cerevisiae* alcohol dehydrogenase/glyceraldehyde-3-phosphate dehydrogenase genes (ADH2/GAP), and the *Saccharomyces cerevisiae* 3-phosphoglycerate kinase gene. Other useful promoters for yeast host cells are described by Romanos et al., 1992, Yeast 8:423-488.

The nucleic acid sequence encoding said conjugate may also be operably connected to a suitable terminator, such as the human growth hormone terminator (Palmiter et al., op. cit.) Preferred terminators for filamentous fungal host cells are obtained from the genes encoding *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

Preferred terminators for yeast host cells are obtained from the genes encoding *Saccharomyces cerevisiae* enolase, *Saccharomyces cerevisiae* cytochrome C (CYC1), or *Saccharomyces cerevisiae* glyceraldehyde-3-phosphate dehydrogenase. Other useful terminators for yeast host cells are described by Romanos et al., 1992, supra.

The vector may further comprise elements such as polyadenylation signals (e.g., from SV 40 or the adenovirus 5 Elb region), transcriptional enhancer sequences (e.g., the SV 40 enhancer) and translational enhancer sequences (e.g., the ones encoding adenovirus VA RNAs). Furthermore, preferred polyadenylation sequences for filamentous fungal host cells are obtained from the genes encoding *Aspergillus oryzae* TAKA amylase, *Aspergillus niger* glucoamylase, *Aspergillus nidulans* anthranilate synthase, and *Aspergillus niger* alpha-glucosidase. Useful polyadenylation sequences for yeast host cells are described by Guo and Sherman, 1995, Molecular Cellular Biology 15:5983-5990.

The recombinant expression vector may further comprise a DNA sequence enabling the vector to replicate in the host cell in question. Examples of such a sequence (when the host cell is a mammalian cell) is the SV 40 or polyoma origin of replication. Examples of bacterial origins of replication are the origins of replication of plasmids pBR322, pUC19, pACYC177, pACYC184, pUB110, pE194, pTA1060, and pAMβ1. Examples of origin of replications for use in a yeast host cell are the 2 micron origin of replication, the combination of CEN6 and ARS4, and the combination of CEN3 and ARS1. The origin of replication may be one having a mutation to make its function temperature-sensitive in the host cell (see, e.g., Ehrlich, 1978, Proc. Natl. Acad. Sci. USA 75:1433).

The vector may also comprise a selectable marker, e.g., a gene the product of which complements a defect in the host cell, such as the gene coding for dihydrofolate reductase (DHFR) or one which confers resistance to a drug, e.g., neomycin, geneticin, ampicillin, or hygromycin. Suitable markers for yeast host cells are ADE2, HIS3, LEU2, LYS2, MET3, TRP1, and URA3. A selectable marker for use in a filamentous fungal host cell may be selected from the group including, but not limited to, amdS (acetamidase), argB (ornithine carbamoyltransferase), bar (phosphinothricin acetyltransferase), hygB (hygromycin phosphotransferase), niaD (nitrate reductase), pyrG (orotidine-5'-phosphate decarboxylase), sC (sulfate adenyltransferase), trpC (anthranilate synthase), and glufosinate resistance markers, as well as equivalents from other species. Preferred for use in an Aspergillus cell are the amdS and pyrG markers of *Aspergillus nidulans* or *Aspergillus oryzae* and the bar marker of *Streptomyces hygroscopicus.* Furthermore, selection may be accomplished by cotransformation, e.g., as described in WO 91/17243, where the selectable marker is on a separate vector.

The procedures used to ligate the nucleic acid sequences coding for the conjugate, the promoter and the terminator, respectively, and to insert them into suitable vectors containing the information necessary for replication, are well known to persons skilled in the art (cf., for instance, Sambrook et al., op.cit.).

The host cell into which the expression vector is introduced may be any cell which is capable of producing the conjugate and is may be a eukaryotic cell, such as invertebrate (insect) cells or vertebrate cells, e.g., *Xenopus laevis* oocytes or mammalian cells, in particular insect and mammalian cells. Examples of suitable mammalian cell lines are the COS (e.g., ATCC CRL 1650), BHK (e.g., ATCC CRL 1632, ATCC CCL 10) or CHO (e.g., ATCC CCL 61) cell lines.

Methods for transfecting mammalian cells and expressing DNA sequences introduced in the cells are described in e.g., Kaufman and Sharp, 1982, J. Mol. Biol. 159:601-621; Southern and Berg, 1982, J. Mol. Appl. Genet. 1:327-341; Loyter et al., 1982, Proc. Natl. Acad. Sci. USA 79:422-426; Wigler et al., 1978, Cell 14:725; Corsaro and Pearson, 1981, Somatic Cell Genetics 7:603, Graham and van der Eb, 1973, Virology 52:456; Fraley et al., 1980, JBC 225:10431; Capecchi, 1980, Cell 22:479; Wiberg et al., 1983,NAR 11:7287; and Neumann et al., 1982, EMBO J. 1:841-845.

The host cell may also be a unicellular pathogen, e.g., a prokaryote, or a non-unicellular pathogen, e.g., a eukaryote. Useful unicellular cells are bacterial cells such as gram positive bacteria including, but not limited to, a *Bacillus* cell, e.g., *Bacillus alkalophilus, Bacillus amyloliquefaciens, Bacillus brevis, Bacillus circulans, Bacillus coagulans, Bacillus lautus, Bacillus lentus, Bacillus licheniformis, Bacillus megaterium, Bacillus stearothermophilus, Bacillus subtilis,* and *Bacillus thuringiensis;* or a Streptomyces cell, e.g., *Streptomyces lividans* or *Streptomyces murinus,* or gram negative bacteria such as *E. coli* and *Pseudomonas* sp. In a preferred embodiment, the bacterial host cell is a *Bacillus lentus, Bacillus licheniformis, Bacillus stearothermophilus* or *Bacillus subtilis* cell. The transformation of a bacterial host cell may, for instance, be effected by protoplast transformation (see, e.g., Chang and Cohen, 1979, Molecular General Genetics 168:111-115), by using competent cells (see, e.g., Young and Spizizin, 1961, Journal of Bacteriology 81:823-829, or Dubnar and Davidoff Abelson, 1971, Journal of Molecular Biology 56:209-221), by electroporation (see, e.g., Shigekawa and Dower, 1988, Biotechniques 6:742-751), or by conjugation (see, e.g., Koehler and Thorne, 1987, Journal of Bacteriology 169:5771-5278).

The host cell may be a fungal cell. "Fungi" as used herein includes the phyla Ascomycota, Basidiomycota, Chytridiomycota, and Zygomycota (as defined by Hawksworth et al., In, Ainsworth and Bisby's Dictionary of The Fungi, 8th edition, 1995, CAB International, University Press, Cambridge, UK) as well as the Oomycota (as cited in Hawksworth et al., 1995, supra, page 171) and all mitosporic fungi (Hawksworth et al., 1995, supra). Representative groups of Ascomycota include, e.g., Neurospora, Eupenicillium (=Penicillium), Emericella (=Aspergillus), Eurotium (=Aspergillus), and the true yeasts listed above. Examples of Basidiomycota include mushrooms, rusts, and smuts. Representative groups of Chytridiomycota include, e.g., Allomyces, Blastocladiella, Coelomomyces, and aquatic fungi. Representative groups of Oomycota include, e.g., Saprolegniomycetous aquatic fungi (water molds) such as Achlya. Examples of mitosporic fungi include Aspergillus, Penicillium, Candida, and Alternaria. Representative groups of Zygomycota include, e.g., Rhizopus and Mucor. The fungal host cell may also be a yeast cell. "Yeast" as used herein includes ascosporogenous yeast (Endomycetales), basidiosporogenous yeast, and yeast belonging to the Fungi Imperfecti (Blastomycetes). The medium used to culture the cells may be any conventional medium suitable for growing mammalian cells, such as a serum-containing or serum-free medium containing appropriate supplements, or a suitable medium for growing insect, yeast or fungal cells. Suitable media are available from commercial suppliers or may be prepared according to published recipes (e.g,. in catalogues of the American Type Culture Collection).

The conjugate or exendin variant produced by the cells may then be recovered from the culture medium by conventional procedures including separating the host cells from the medium by centrifugation or filtration, precipitating the proteinaceous components of the supernatant or filtrate by means of a salt, e.g., ammonium sulphate, purification by a variety of chromatographic procedures, e.g., ion exchange chromatography, affinity chromatography, or the like.

The lipophilic substituent(s) may be attached to the peptide of the present invention using procedures known in the art. In one embodiment, the lipophilic substituent may be attached by incorporating an amino acid with the lipophilic substituent already attached in the standard synthesis method (see, for example, synthesis of compounds 7, 8 and 9 in the Examples section). Alternatively, the substituent may be attached after the peptide has been synthesized and isolated as, for example, described in WO98/08871.

The invention is further illustrated by the following examples.

### EXAMPLES

### Peptide Synthesis

### General Procedures

### Apparatus and synthetic strategy

Peptides are synthesized batchwise in a polyethylene vessel equipped with a polypropylene filter for filtration using 9-fluorenylmethyloxycarbonyl (Fmoc) as the N-α-amino protecting group and suitable common protection groups for side-chain functionalities (Dryland et al., 1986, J. Chem. Soc., Perkin Trans. 1:125-137).

### Solvents

Solvent DMF (*N,N*-dimethylformamide, Riedel de-Häen, Germany) is purified by passing it through a column packed with a strong cation exchange resin (Lewatit S 100 MB/H strong acid, Bayer AG Leverkusen, Germany) and analysed for free amines prior to use by addition of 3,4-dihydro-3-hydroxy-4-oxo-1,2,3-benzotriazine (Dhbt-OH) giving rise to a yellow color (Dhbt-O-anion) if free amines are present. Solvent DCM (dichloromethane, analytical grade, Riedel de-Häen, Germany) is used directly without purification. THF (tetrahydrofuran, analytical grade, Riedel de-Haen, Germany) is used directly without further purification.

### Amino acids

Fmoc-protected amino acids are purchased from MilliGen (UK) and from PerSeptive Biosystems GmbH Hamburg, Germany in suitable side-chain protected forms. FmocLys(palmitoyl)-OH is purchased from Bachem (Switzerland).

### Linker

(4-hydroxymethylphenoxy)acetic acid (HMPA), Novabiochem, Switzerland is coupled to the resin either as a preformed or *in situ* generated 1-hydroxybenzotriazole (HObt) ester by means of DIC.

### Coupling reagents

Coupling reagent diisopropylcarbodiimide (DIC) is purchased from (Riedel de-Häen, Germany) and distilled prior to use, dicyclohexylcarbodiimide (DCC) is purchased from Merck-Schuchardt, München, Germany, and purified by distillation.

### Solid supports

Peptides synthesized according to the Fmoc-strategy are synthesized on the following types of solid support using 0.05 M or higher concentrations of Fmoc-protected activated amino acid in DMF. TentaGel S resins 0.22-0.31 mmol/g (TentaGel S-Ram, TentaGel S RAM-Lys(Boc)Fmoc; Rapp polymere, Germany).

### Catalysts and other reagents

Diisopropylethylamine (DIEA) is purchased from Aldrich, Germany, and ethylenediamine from Fluka, piperidine and pyridine from Riedel-de Häen, Frankfurt, Germany. 4-(N,N-dimethylamino)pyridine (DMAP) is purchased from Fluka, Switzerland and used as a catalyst in coupling reactions involving symmetrical anhydrides. Ethanedithiol is purchased from Riedel-de Häen, Frankfurt, Germany. 3,4-dihydro-3-hydroxy-4-oxo-1,2,3-benzotriazine (Dhbt-OH) and 1-hydroxybenzotriazole (HObt) are obtained from Fluka, Switzerland.

### Coupling procedures

The first amino acid is coupled as a symmetrical anhydride in DMF generated from the appropriate N-α-protected amino acid by means of DIC or DCC. The following amino acids are coupled as preformed HObt esters made from appropriate N-α-protected amino acids and HObt by means of DIC in DMF. Acylations are checked by the ninhydrin test performed at 80°C in order to prevent Fmoc deprotection during the test (Larsen, B. D. and Holm, A., 1994, Int. J. Peptide Protein Res. 43:1-9).

### Coupling as HObt-ester

*Method a.* 3 eq. N-α-amino protected amino acid is dissolved in DMF together with 3 eq. HObt and 3 eq DIC. The solution is left at r.t. for 10 minutes and then added to the resin, which had been washed with a solution of 0.2% Dhbt-OH in DMF prior to the addition of the preactivated amino acid.

*Method b.* 3 eq. N-α-amino protected amino acid is dissolved in DMF together with 3 eq. HObt. 3 eq DIC are added just prior to use. The final solution is added to the resin.

### Preformed symmetrical anhydride

6 eq. N-α-amino protected amino acid is dissolved in DCM and cooled to 0°C. DCC or DIC (3 eq.) is added and the reaction continued for 10 min. The solvent is removed *in vacuo* and the residue dissolved in DMF. The DMF-solution is filtered in case of using DCC and immediately added to the resin followed by 0.1 eq. of DMAP.

### Deprotection of the N-α-amino Fmoc protecting group

Deprotection of the Fmoc group is performed by treatment with 20% piperidine in DMF (1 x 5 and 1 x 10 min.), followed by wash with DMF until no yellow colour (Dhbt-O-) could be detected after addition of Dhbt-OH to the drained DMF.

### Cleavage of peptide from resin with acid

*Method a.* Peptides are cleaved from the resins by treatment with 95% trifluoroacetic acid (TFA, Riedel-de Häen, Frankfurt, Germany)-water v/v or with 95% TFA and 5% ethanedithiol v/v at r.t. for 2 h. The filtered resins are washed with 95% TFA-water and filtrates and washings are diluted by adding 10% acetic acid. The resulting mixture is extracted 3 times with ether and finally freeze dried. The crude freeze dried product is analysed by high-performance liquid chromatography (HPLC) and identified by mass spectrometry (MS).

### Batchwise peptide synthesis on TentaGel S-RAM

TentaGel S-RAM resin (100-1000 mg, 0.22-0.31 mmol/g) is placed in a polyethylene vessel equipped with a polypropylene filter for filtration. The resin is swelled in DMF (5-10 ml), and the Fmoc group is removed according to the procedure described above. The following amino acids according to the sequence are coupled as Fmoc-protected HObt esters (3 eq.) generated *in situ* by means of DIC as described above. The couplings are continued for 3 h, unless otherwise specified. The resin is drained and washed with DMF (4 x 5-10 ml, 2 min each) in order to remove excess reagent. All acylations are checked by the ninhydrin test performed at 80°C. After completion of the synthesis, the peptide-resin is washed with DMF (3 x 5-10 ml, 5 min each), DCM (3 x 5-10 ml, 1 min each) and finally diethyl ether (3 x 5-10 ml, 1 min each) and dried *in vacuo.*

### HPLC conditions

Isocratic HPLC analysis is preformed on a Shimadzu system consisting of an LC-6A pump, an MERCK HITACHI L-4000 UV detector operated at 215 nm and a Rheodyne 7125 injection valve with a 20 µl loop. The column used for isocratic analysis is a Spherisorb ODS-2 (100 x 3 mm; 5-µm particles) (MicroLab, Aarhus, Denmark).

HPLC analysis using gradients is performed on a MERCK-HITACHI L-6200 Intelligent pump, an MERCK HITACHI L-4000 UV detector operated at 215 nm and a Rheodyne 7125 injection valve with a 20 µl loop, or on a Waters 600 E instrument equipped with a Waters 996 photodiode array detector. The columns used are a Rescorce™ RPC 1 ml (Waters) or a LiChroCART 125-4, LiChrospher 100 RP-18 (5 µm) (Merck).

Buffer A is 0.1 vol % TFA in water and buffer B 90 vol% acetonitrile, 9.9 vol% water and 0.1 vol% TFA. The buffers are pumped through the columns at a flow rate of 1.3-1.5 ml/min using either of the following gradients for peptide analysis 1) Linear gradient from 0% - 100% B (30 min) or 2) 0% B (2 min) linear gradient from 0-50% B (23 min) 50-100% B (5 min).

For Preparative HPLC, purification is performed on a Waters 600 E instrument equipped with a Waters 996 photodiode array detector. The column used is a Waters Delta-Pak C-18 15 µm, 100 Å, 25 x 100 mm. Gradient "2)" is used with a flow rate of 9 ml/min.

### Mass spectroscopy

Mass spectra are obtained on a Finnigan Mat LCQ instrument equipped with an electrospray (ESI) probe (ES-MS) and on a TofSpec E, Fisons Instrument (MALDI-TOF) using β-cyano-*p*-hydroxycinnamic acid as matrix.

### Peptide synthesis of individual peptides

### 1. Peptide synthesis ofH-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser-NH₂ (des Pro³⁶-exendin-4-NH₂) on TentaGel S-RAM

Dry TentaGel S-RAM resin (0.25 mmol/g, 1500 mg) is placed in a polyethylene vessel equipped with a polypropylene filter for filtration and swelled for two hours in DMF (5 ml). The Fmoc group is removed according to the procedure described above, and the peptide according to the sequence is assembled as described under "Batchwise peptide synthesis on TentaGel S-RAM resins". After completion of the synthesis, the peptide-resin is washed with DMF (3x5 ml, 1 min each), DCM (3x5 ml, 1 min each), diethyl ether (3x5 ml, 1 min each) and dried *in vacuo.*

The peptide is cleaved from the resin according to *method a* as described above and freeze dried from acetic acid. The crude peptide is purified by preparative HPLC using the procedure described above. The purified product is found to be homogeneous and the purity is found to be better than 95%. The identity of the peptide is confirmed by ES-MS. Yield 18.3%.

### 2. Peptide synthesis of H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser- Lys₆-NH₂ (des-Pro³⁶-exendin-4-Lys₆-NH₂) on TentaGel S-RAM-Lys(Boc)Fmoc

Dry TentaGel S-RAM-Lys(Boc)Fmoc resin (0.22 mmol/g, 1500 mg) is placed in a polyethylene vessel equipped with a polypropylene filter for filtration and swelled for two hours in DMF (5 ml). The Fmoc group on the first lysine is removed as described above and the synthesis is continued until finishing the peptide sequence as described under "Batchwise peptide synthesis on TentaGel S-Ram-Lys(Boc)Fmoc". After completion of the synthesis, the peptide-resin is washed with DMF (3x5 ml, 1 min each), DCM (3x5 ml, 1 min each), diethyl ether (3x5 ml, 1 min each) and dried *in vacuo.*

The peptide is cleaved from the resin according to *method a* as described above and freeze dried from acetic acid. The crude freeze dried product is purified by preparative HPLC using the procedure described above. The purified product is found to be homogeneous and the purity is found to be better than 95%. The identity of the peptide is confirmed by ES-MS. Yield 22.1%.

### 3. Peptide synthesis of H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser -NH₂(exendin-4-NH₂) on TentaGel S-RAM

Dry TentaGel S-RAM resin (0.25 mmol/g, 1000 mg) is placed in a polyethylene vessel equipped with a polypropylene filter for filtration and swelled for two hours in DMF (5 ml). The Fmoc group is removed according to the procedure described above, and the peptide according to the sequence is assembled as described under "Batchwise peptide synthesis on TentaGel S-RAM resins". After completion of the synthesis, the peptide-resin is washed with DMF (3x5 ml, 1 min each), DCM (3x5 ml, 1 min each), diethyl ether (3x5 ml, 1 min each) and dried *in vacuo.*

The peptide is cleaved from the resin according to *method a* as described above and freeze dried from acetic acid. The crude peptide is purified by preparative HPLC using the procedure described above. The purified product is found to be homogeneous and the purity is found to be better than 90%. The identity of the peptide is confirmed by ES-MS. Yield 17%.

### 4. Peptide synthesis of H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser- Lys₆-NH₂ (exendin-4-Lys₆-NH₂) on TentaGel S-RAM-Lys(Boc)Fmoc

Dry TentaGel S-RAM-Lys(Boc)Fmoc resin (0.22 mmol/g, 1000 mg) is placed in a polyethylene vessel equipped with a polypropylene filter for filtration and swelled for two hours in DMF (5 ml). The Fmoc group on the first lysine is removed as described above and the synthesis is continued until finishing the peptide sequence as described under "Batchwise peptide synthesis on TentaGel S-Ram-Lys(Boc)Fmoc". After completion of the synthesis, the peptide-resin is washed with DMF (3x5 ml, 1 min each), DCM (3x5 ml, 1 min each), diethyl ether (3x5 ml, 1 min each) and dried *in vacuo.*

The peptide is cleaved from the resin according to *method a* as described above and freeze dried from acetic acid. The crude freeze dried product is purified by preparative HPLC using the procedure described above. The purified product is found to be homogeneous and the purity is found to be better than 90%. The identity of the peptide is confirmed by ES-MS. Yield 20.5%.

### 5. Peptide synthesis of H- His-Gly-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-NH₂ (Gly⁸-GLP1-(7-36)(Human)-NH₂) on TentaGel S-RAM

Dry TentaGel S-RAM resin (0.25 mmol/g, 1000 mg) is placed in a polyethylene vessel equipped with a polypropylene filter for filtration and swelled for two hours in DMF (5 ml). The Fmoc group is removed according to the procedure described above, and the peptide according to the sequence is assembled as described under "Batchwise peptide synthesis on TentaGel S-RAM resins". After completion of the synthesis, the peptide-resin is washed with DMF (3x5 ml, 1 min each), DCM (3x5 ml, 1 min each), diethyl ether (3x5 ml, 1 min each) and dried *in vacuo.*

The peptide is cleaved from the resin according to *method a* as described above and freeze dried from acetic acid. The crude peptide is purified by preparative HPLC using the procedure described above. The purified product is found to be homogeneous and the purity is found to be better than 95%. The identity of the peptide is confirmed by ES-MS. Yield 9%.

### 6. Peptide synthesis of H-His-Gly-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Lys₆-NH₂ (Gly⁸-GLP1-(7-36) (Human)-Lys₆-NH₂) on TentaGel S-RAM-Lys(Boc)Fmoc

Dry TentaGel S-RAM-Lys(Boc)Fmoc resin (0.22 mmol/g, 1000 mg) is placed in a polyethylene vessel equipped with a polypropylene filter for filtration and swelled for two hours in DMF (5 ml). The Fmoc group on the first lysine is removed as described above and the synthesis is continued until finishing the peptide sequence as described under "Batchwise peptide synthesis on TentaGel S-Ram-Lys(Boc)Fmoc". After completion of the synthesis, the peptide-resin is washed with DMF (3x5 ml, 1 min each), DCM (3x5 ml, 1 min each), diethyl ether (3x5 ml, 1 min each) and dried *in vacuo.*

The peptide is cleaved from the resin according to *method a* as described above and freeze dried from acetic acid. The crude freeze dried product is purified by preparative HPLC using the procedure described above. The purified product is found to be homogeneous and the purity is found to be better than 95%. The identity of the peptide is confirmed by ES-MS. Yield 11.7%.

### 7. Peptide synthesis of H- His-Gly-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Lys(palmitoyl)-Lys₆-NH₂ (Gly⁸)(Lys³⁷(palmitoyl))-GLP1-(7-36)(Human)-Lys₇-NH₂) on TentaGel S-RAM-Lys(Boc)Fmoc

Dry TentaGel S-RAM-Lys(Boc)Fmoc resin (0.22 mmol/g, 1000 mg) is placed in a polyethylene vessel equipped with a polypropylene filter for filtration and swelled for two hours in DMF (5 ml). The Fmoc group on the first lysine is removed as described above and the synthesis is continued until finishing the peptide sequence as described under "Batchwise peptide synthesis on TentaGel S-Ram-Lys(Boc)Fmoc". The reagent Fmoc-Lys(palmitoyl)-OH is coupled in a slightly modified manner due to its poor solubility in DMF. Approximately 400 mg of Fmoc-Lys(palmitoyl)-OH is dissolved in approximately 6 ml THF rather than DMF. After completion of the synthesis, the peptide-resin is washed with DMF (3x5 ml, 1 min each), DCM (3x5 ml, 1 min each), diethyl ether (3x5 ml, 1 min each) and dried *in vacuo.*

The peptide is cleaved from the resin according to *method b* as described above and freeze dried from acetic acid. The crude freeze dried product is purified by preparative HPLC using the procedure described above. The purified product is found to be homogeneous and the purity is found to be better than 95%. The identity of the peptide is confirmed by ES-MS. Yield 9.3%.

### 8. Peptide synthesis of H- His-Gly-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val- Lys(palmitoyl)-Gly-Arg-Lys₆-NH2 (Gly⁸)(Lys³⁴(palmitoyl))-GLP1-(7-36)(Human)-Lys₆-NH₂) on TentaGel S-RAM-Lys(Boc)Fmoc

Dry TentaGel S-RAM-Lys(Boc)Fmoc resin (0.22 mmol/g, 1000 mg) is placed in a polyethylene vessel equipped with a polypropylene filter for filtration and swelled for two hours in DMF (5 ml). The Fmoc group on the first lysine is removed as described above and the synthesis is continued until finishing the peptide sequence as described under "Batchwise peptide synthesis on TentaGel S-Ram-Lys(Boc)Fmoc". The reagent Fmoc-Lys(palmitoyl)-OH is coupled in a slightly modified manner due to its poor solubility in DMF. Approximately 400 mg of Fmoc-Lys(palmitoyl)-OH is dissolved in approximately 6 ml THF rather than DMF. After completion of the synthesis, the peptide-resin is washed with DMF (3x5 ml, 1 min each), DCM (3x5 ml, 1 min each), diethyl ether (3x5 ml, 1 min each) and dried *in vacuo.*

The peptide is cleaved from the resin according to *method a* as described above and freeze dried from acetic acid. The crude freeze dried product is purified by preparative HPLC using the procedure described above. The purified product is found to be homogeneous and the purity is found to be better than 90%. The identity of the peptide is confirmed by ES-MS. Yield 7.5%.

### 9. Peptide synthesis of H-His-Gly-Glu-Gly-Thr-Phe-Thr-Ser-Asp-Val-Ser-Ser-Tyr-Leu-Glu-Gly-Gln-Ala-Ala-Lys(palmitoyl)-Glu-Phe-Ile-Ala-Trp-Leu-Val-Lys-Gly-Arg-Lys₆-NH2 (Gly⁸)(Lys²⁶(palmitoyl))-GLP1-(7-36)(Human)-Lys₆-NH2) on TentaGel S-RAM-Lys(Boc)Fmoc

Dry TentaGel S-RAM-Lys(Boc)Fmoc resin (0.22 mmol/g, 1000 mg) is placed in a polyethylene vessel equipped with a polypropylene filter for filtration and swelled for two hours in DMF (5 ml). The Fmoc group on the first lysine is removed as described above and the synthesis is continued until finishing the peptide sequence as described under "Batchwise peptide synthesis on TentaGel S-Ram-Lys(Boc)Fmoc". The reagent Fmoc-Lys(palmitoyl)-OH is coupled in a slightly modified manner due to its poor solubility in DMF. Approximately 400 mg of Fmoc-Lys(palmitoyl)-OH is dissolved in approximately 6 ml THF rather than DMF. After completion of the synthesis, the peptide-resin is washed with DMF (3x5 ml, 1 min each), DCM (3x5 ml, 1 min each), diethyl ether (3x5 ml, 1 min each) and dried *in vacuo.*

The peptide is cleaved from the resin according to *method a* as described above and freeze dried from acetic acid. The crude freeze dried product is purified by preparative HPLC using the procedure described above. The purified product is found to be homogeneous and the purity is found to be better than 90%. The identity of the peptide is confirmed by ES-MS. Yield 8.9%.

### In vivo Studies

### Studies with des Pro³⁶-exendin-4-NH₂ and des Pro³⁶-exendin-4-Lys6,-NH₂, Gly⁸-GLP1-(7-36)(Human)-NH₂ and (Gly⁸-GLP1-(7-36)(Human)-Lys₆-NH₂ and Gly⁸Lys³⁷(palmitoyl)-GLP1-(7-36)(Human)-Lys₇-NH₂

Various concentrations of each peptide are administered orally and intraperitoneally to *ob/ob* mice to determine if these compounds affect blood glucose levels.

### Materials and methods

### Animals and glucose analysis

Forty female diabetic *ob/ob* mice (Umeå strain, Bomholtgaard), which are obese due to a dominant mutant leptin gene (Tomita et al., 1992, Pancreas 7:367-375) are housed (3 mice/cage) under controlled ambient conditions following a 12:12-h light:dark cycle with light on at 6 am, and fed standard Altromin no 1324 diet with free access to tap water.

The animals are handled for 3 days before each experiment was performed to reduce blood glucose excursions when arousing the animals during the experiment.

On the day of each experiment, blood is taken from the tail vein 2-3 hours after the lights-on and blood glucose is measured. The blood glucose level between animals varied between normoglucaemic and major hyperglycemic states. Animals with the lowest glucose levels (< 5.6 mM) are excluded from the study. The remaining animals are sorted into groups based on their glucose levels and keeping mean concentrations constant between the groups. Approximately 60 min after the initial blood sampling, the animals are injected with the compounds. Blood glucose levels are followed throughout up to 8 hours after the treatment (t=60 min, t=120 min, t=240 min).

Blood is taken from the tail vein and dropped on the strip for analysis. Whole blood glucose (mM) concentration (PG) is analysed by the immobilised glucose oxidase method using a drop of blood (< 5 µl; Elite autoanalyser, Bayer, Denmark) following the manufacturer's manual.

### Peptides and other materials

des Pro³⁶-exendin-4-NH₂ and the same peptide, but with an additional sequence, Lys₆, attached at the C-terminal, des Pro³⁶-exendin-4-Lys6-NH₂, Gly⁸-GLP1-(7-36)(Human)-NH2 and the same peptide, but with an additional sequence, Lys₆, attached at the C-terminal, Gly⁸-GLP1-(7-36)(Human)- Lys6-NH2 and Gly⁸Lys³⁷(palmitoyl)-GLP1-(7-36)(Human)- Lys₇-NH₂, are synthesized using methods described above.

Solutions are prepared on the morning of dosing, immediately before the animals are administered. The same solutions are used for both peroral and interperitoneal administration. All peptides are dissolved in sterile isotonic NaCl and given in a volume of 0.2 ml.

### Experiments

All experiments are carried out in the same mice to compare the active doses of the peptides shown in Table I. Blood sampling is performed as described above and the animals are administered with the doses shown in Table II. As negative control, saline is administered perorally, and as positive control a solution of the peptide is given intraperitoneally.

**TABLE I**

| Number | Compound |
|---|---|
| ZS42-0009 | des Pro³⁶-exendin-4-NH₂ |
| ZS42-0010 | des Pro³⁶-exendin-4-Lys₆-NH2 |
| ZS42-0032 | Gly⁸-GLP1-(7-36)(Human)-NH2 |
| ZS42-0020 | Gly⁸-GLP1-(7-36)(Human)-Lys₆-NH2 |
| ZS42-0019 | Gly⁸Lys³⁷(palmitoyl)-GLP1-(7-36)(Human)-Lys₇-NH2 |

**TABLE II**

| Compound | **Group 1 Dose Peroral** µg/mouse | **Group 2 Dose peroral** µg/mouse | **Group 3 Dose peroral** µg/mouse | **Group 4 Dose peroral** µg/mouse | **Group 5 Dose Peroral** µl/mouse Isotonic saline | **Group 6 Dose i.p** µg/mouse |
|---|---|---|---|---|---|---|
| ZS42-0009 | 400 | 40 | 4 | 0.4 | 200 µl | 40 |
| ZS42-0010 | 1000 | 100 | 10 | 1 | 200 µl | 100 |
| ZS42-0032 | 1000 | 100 | 10 | 1 | 200 µl | 100 |
| ZS42-0020 | 1000 | 100 | 10 | 1 | 200 µl | 100 |
| ZS42-0019 | 1000 | 100 | 10 | 1 | 200 µl | 100 |

### Analysis of data

Group data were summarised as the mean ± SEM of the individual results in each treatment group. In order to analyse the effects of the compounds, the absolute and the relative (% of t = 0) difference from baseline was calculated for each time point.

### Results

**TABLE III**

| | **0** | **1 hour** | **2 hours** | **4 hours** |
|---|---|---|---|---|
| ZS42-0009-saline | **100** | **103** | **107** | **92** |
| ZS42-0009-400 ug po | **100** | **93** | **88** | **93** |
| ZS42-0009-40 ug po | **100** | **89** | **89** | **91** |
| ZS42-0009-4 ug po | **100** | **105** | **88** | **91** |
| ZS42-0009-0.4 ug po | **100** | **106** | **103** | **100** |
| ZS42-0009-40 ug ip | **100** | **68** | **69** | **74** |
| | | | | |
| ZS42-0010-saline | **100** | **100** | **112** | **114** |
| ZS42-0010-1000 ug po | **100** | **67** | **69** | **64** |
| ZS42-0010 100 ug po | **100** | **78** | **71** | **72** |
| ZS42-0010 10 ug po | **100** | **86** | **72** | **72** |
| ZS42-0010 1 ug po | **100** | **112** | **101** | **96** |
| ZS42-0010 100 ug ip | **100** | **75** | **67** | **63** |
| | | | | |
| ZS42-0032-saline | **100** | **95** | **87** | **100** |
| ZS42-0032 -1000 ug po | **100** | **87** | **105** | **94** |
| ZS42-0032 -100 ug po | **100** | **118** | **111** | **92** |
| ZS42-0032 -10 ug po | **100** | **101** | **94** | **104** |
| ZS42-0032 -1 ug po | **100** | **94** | **89** | **96** |
| ZS42-0032 -100 ug ip | **100** | **70** | **60** | **81** |
| | | | | |
| ZS42-0020-saline | **100** | **102** | **94** | **79** |
| ZS42-0020 -1000 ug po | **100** | **128** | **72** | **78** |
| ZS42-0020 -100 ug po | **100** | **72** | **70** | **58** |
| ZS42-0020 -10 ug | **100** | **98** | **95** | **81** |
| po | | | | |
| ZS42-0020 -1 ug po | **100** | **99** | **89** | **84** |
| ZS42-0020 100 ug ip | **100** | **83** | **58** | **56** |
| | | | | |
| ZS42-0019 -saline | **100** | **90** | **86** | **103** |
| ZS42-0019 -1000 ug po | **100** | **73** | **75** | **67** |
| ZS42-0019 -100 ug po | **100** | **97** | **140** | **107** |
| ZS42-0019 -10 ug po | **100** | **90** | **120** | **126** |
| ZS42-0019 -1 ug po | **100** | **111** | **133** | **114** |
| ZS42-0019 - 100 ug ip | **100** | **63** | **50** | **52** |

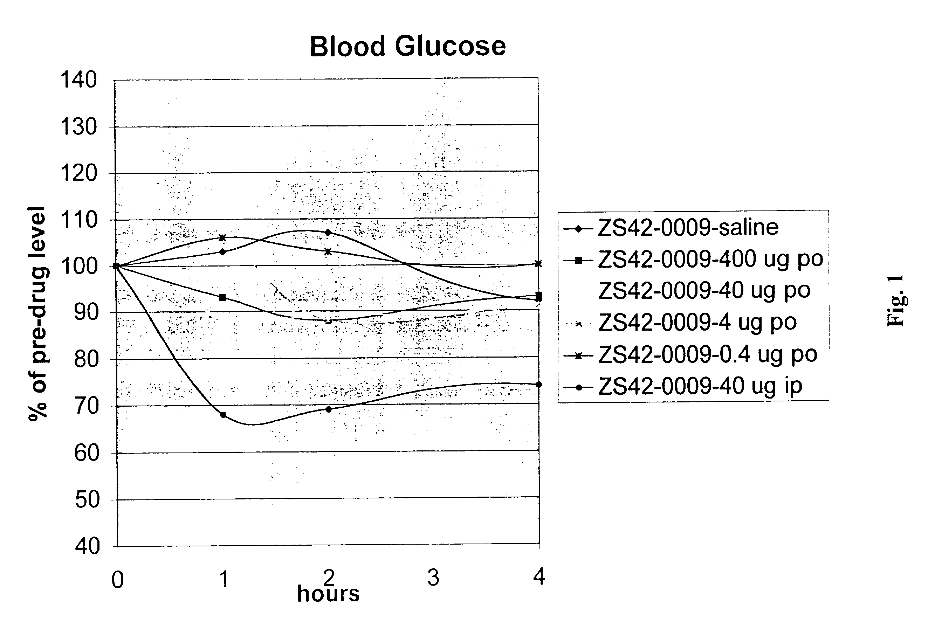

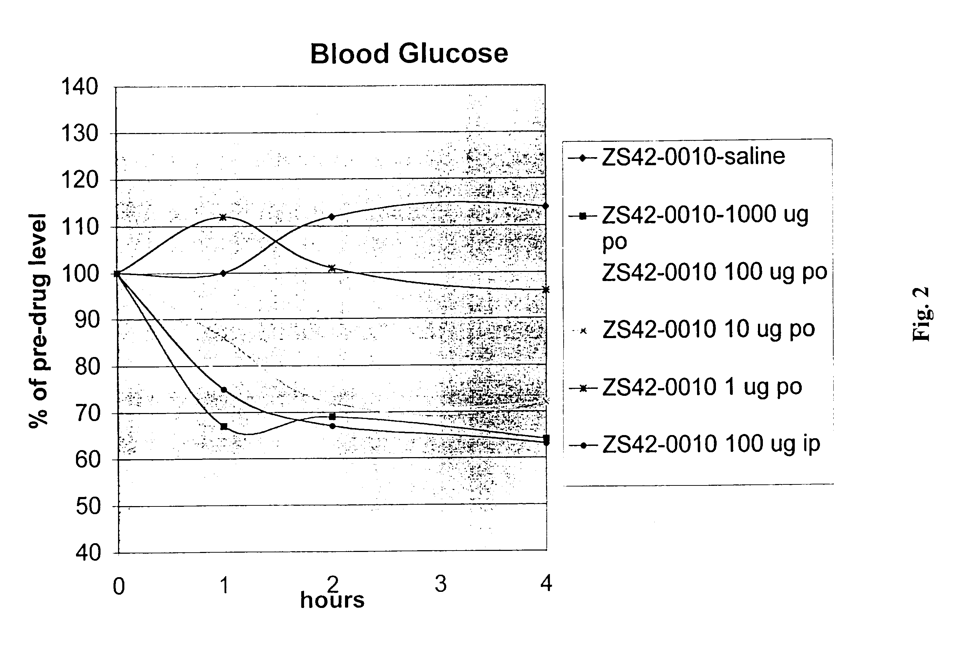

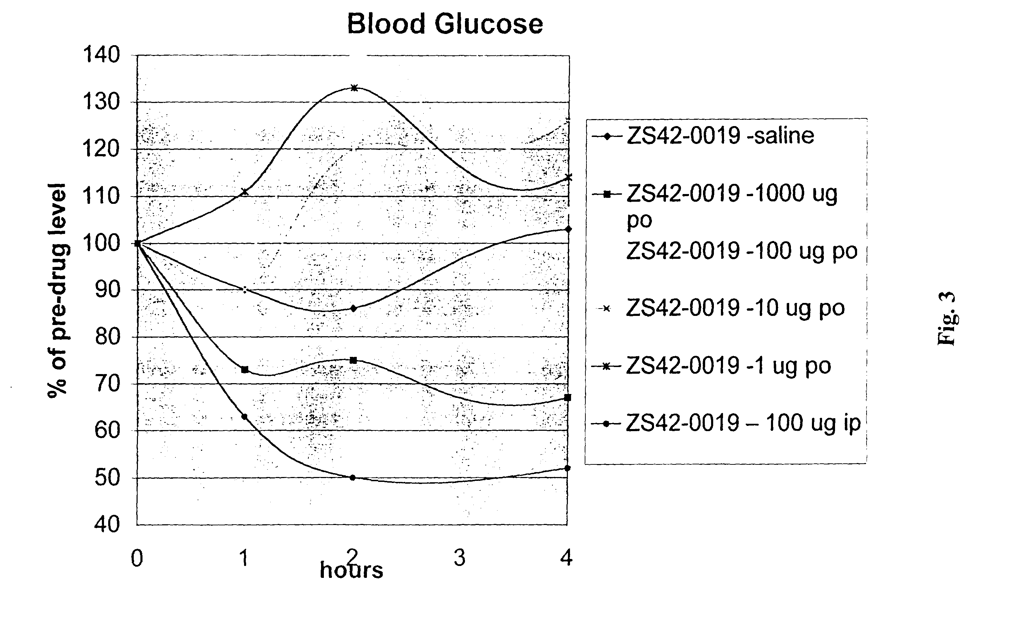

The results obtained are shown in Table III and in Figures 1-3.

### Discussion

These results show that all of the compounds have an effect in lowering blood glucose levels when given intraperitoneally. The potency of des Pro³⁶-exendin-4-NH₂ and des Pro³⁶-exendin-4-Lys₆-NH₂ when given intraperitoneally is shown to be very similar to exendin-4-NH2 itself administered in the same way.

For des Pro³⁶-exendin-4-NH₂ there is no effect in lowering blood glucose levels up to a dose of 400 µg/mouse when the compound is administered perorally, whereas for the same compound with the addition of the Lys6 fragment there is activity seen at a dose of 10 µ/mouse. This indicates that the minimum effective dose of the des Pro³⁶-exendin-4-Lys₆-NH2 is at least 40 times lower than for des Pro³⁶-exendin-4-NH₂.

For Gly⁸-GLP1-(7-36)(Human)-NH2, there is no effect in lowering blood glucose levels up to a dose of 1000 µg/mouse when administered perorally, whereas for the same compound with the addition of the Lys₆ fragment there is activity seen at a dose of 100 µ/mouse. The minimum effective dose of Gly⁸-GLP1-(7-36)(Human)-Lys₆-NH₂ is therefore at least 10 times lower than for Gly⁸-GLP1-(7-36)(Human)-NH₂.
Gly⁸Lys³⁷(palmitoyl)-GLP1-(7-36)(Human)- Lys₇-NH2 has been shown to be active after peroral administration at a dose of 1000 µg/mouse.

These results show that the attachment of the sequence Z has no significant effect on the potency of the various peptides when administered interperetoneally while significantly enhancing the potency of the compound when administered perorally.

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims.

Various references are cited herein, the disclosure of which are incorporated by reference in their entireties.

## Claims

1. A variant of a parent exendin, wherein said parent exendin has an amino acid sequence having at least an 90% homology to exendin-4 and wherein said variant lowers the blood glucose level and binds to a GLP-1 receptor in a mammal and has at least one modification selected from the group consisting of:
(a) between one and five deletions at positions 34-39, and
(b) contains a Lys at position 40 having a lipophilic substituent bound to the epsilon-amino group of Lys via an amide bond.

2. The variant according to claim 1, wherein said variant is selected from the group consisting of des Ser³⁹-exendin-4-NH₂, des Pro³⁶-exendin-4-NH₂, des Ala³⁵-exendin-4-NH₂ and des Gly³⁴-exendin-4-NH₂, des Ser³⁹- (Lys⁴⁰ (palmitoyl) exendin-4-NH₂, des Gly³⁴- (Lys⁴⁰ (palmitoyl)) exendin-4-NH₂, and des Ala³⁵- (Lys⁴⁰ (palmitoyl) exendin-4-NH₂.

3. A pharmaceutical composition comprising a variant of a parent exendin as defined in any of the claims 1-2, and a physiologically acceptable carrier.

4. Use of a variant of claims 1-2 for the manufacture of a pharmaceutical composition for use in treatment of diabetes type 1 or type 2, obesity, eating disorders, hyperglycemia, metabolic disorders, bone disease, gastric disease and insulin resistance syndrome.

5. A peptide conjugate comprising a peptide X which reduces the blood glucose level in a mammal, wherein X is:
(a) an exendin having at least 90% homology to exendin-4;
(b) a variant of said exendin wherein said variant comprises a modification selected from the group consisting of between one and five deletions at positions 34-39 and contains a Lys at position 40 having a lipophilic substituent; or
(c) GLP-1 (7-36) or GLP-1 (7-37) having at least one modification selected from the group consisting of:
(i) substitution of D-alanine, glycine or alpha-amino isobutyric acid for alanine at position 8 and
(ii) a lipophilic substituent
and Z, a peptide sequence, of 4-20 amino acid units covalently bound to X, wherein each amino acid unit in said peptide sequence, Z is selected from the group consisting of Ala, Leu, Ser, Thr, Tyr, Asn, Gln, Asp, Glu, Lys, Arg, His, Met, Orn, and amino acid units of the general formula I
-NH-C(R¹)(R²)-C(=O)- (I)
wherein R¹ and R² are selected from the group consisting of hydrogen, C₁₋₆-alkyl, phenyl, and phenyl-methyl, wherein C₁₋₆-alkyl is optionally substituted with from one to three substituents selected from halogen, hydroxy, amino, cyano, nitro, sulfono, and carboxy, and phenyl and phenyl-methyl is optionally substituted with from one to three substituents selected from C₁₋₆-alkyl, C₂₋₆-alkenyl, halogen, hydroxy, amino, cyano, nitro, sulfono, and carboxy, or R¹ and R² together with the carbon atom to which they are bound form a cyclopentyl, cyclohexyl, or cycloheptyl ring, e.g. 2,4-diaminobutanoic acid and 2,3-diaminopropanoic acid; and
wherein the ratio between the minimum effective oral dose of said peptide conjugate and the minimum effective oral dose of the peptide, X is at least 1:5.

6. The peptide conjugate according to claim 5, wherein Z is 
Lys-Lys-Lys-Lys, Xaa-Lys-Lys-Lys, Lys-Xaa-Lys-Lys, Lys-Lys-Xaa-Lys, Lys-
Lys-Lys-Xaa, Xaa-Xaa-Lys-Lys, Xaa-Lys-Xaa-Lys, Xaa-Lys-Lys-Xaa, Lys-Xaa-
Xaa-Lys, Lys-Xaa-Lys-Xaa, Lys-Lys-Xaa-Xaa, Xaa-Xaa-Xaa-Lys, Xaa-Xaa-Lys-
Xaa, Xaa-Lys-Xaa-Xaa, Lys-Xaa-Xaa-Xaa, Xaa-Xaa-Xaa-Xaa, Lys-Lys-Lys-Lys-
Lys, Xaa-Lys-Lys-Lys-Lys, Lys-Xaa-Lys-Lys-Lys, Lys-Lys-Xaa-Lys-Lys, Lys-
 
Lys-Lys-Xaa-Lys, Lys-Lys-Lys-Lys-Xaa, Xaa-Xaa-Lys-Lys-Lys, Xaa-Lys-Xaa-
Lys-Lys, Xaa-Lys-Lys-Xaa-Lys, Xaa-Lys-Lys-Lys-Xaa, Lys-Xaa-Xaa-Lys-Lys,
Lys-Xaa-Lys-Xaa-Lys, Lys-Xaa-Lys-Lys-Xaa, Lys-Lys-Xaa-Xaa-Lys, Lys-Lys-
Xaa-Lys-Xaa, Lys-Lys-Lys-Xaa-Xaa, Lys-Lys-Xaa-Xaa-Xaa, Lys-Xaa-Lys-Xaa-
Xaa, Lys-Xaa-Xaa-Lys-Xaa, Lys-Xaa-Xaa-Xaa-Lys, Xaa-Lys-Lys-Xaa-Xaa, Xaa-
Lys-Xaa-Xaa-Lys, Xaa-Xaa-Lys-Lys-Xaa, Xaa-Xaa-Lys-Xaa-Lys, Xaa-Xaa-Xaa-
Lys-Lys, Lys-Xaa-Xaa-Xaa-Xaa, Xaa-Lys-Xaa-Xaa-Xaa, Xaa-Xaa-Lys-Xaa-Xaa,
Xaa-Xaa-Xaa-Lys-Xaa, Xaa-Xaa-Xaa-Xaa-Lys, Xaa-Xaa-Xaa-Xaa-Xaa, Lys-Lys-
Lys-Lys-Lys-Lys, Xaa-Lys-Lys-Lys-Lys-Lys, Lys-Xaa-Lys-Lys-Lys-Lys, Lys-
Lys-Xaa-Lys-Lys-Lys, Lys-Lys-Lys-Xaa-Lys-Lys, Lys-Lys-Lys-Lys-Xaa-Lys,
Lys-Lys-Lys-Lys-Lys-Xaa, Xaa-Xaa-Lys-Lys-Lys-Lys, Xaa-Lys-Xaa-Lys-Lys-
Lys, Xaa-Lys-Lys-Xaa-Lys-Lys, Xaa-Lys-Lys-Lys-Xaa-Lys, Xaa-Lys-Lys-Lys-
Lys-Xaa, Lys-Xaa-Xaa-Lys-Lys-Lys, Lys-Xaa-Lys-Xaa-Lys-Lys, Lys-Xaa-Lys-
Lys-Xaa-Lys, Lys-Xaa-Lys-Lys-Lys-Xaa, Lys-Lys-Xaa-Xaa-Lys-Lys, Lys-Lys-
Xaa-Lys-Xaa-Lys, Lys-Lys-Xaa-Lys-Lys-Xaa, Lys-Lys-Lys-Xaa-Xaa-Lys, Lys-
Lys-Lys-Xaa-Lys-Xaa, Lys-Lys-Lys-Lys-Xaa-Xaa, Xaa-Xaa-Xaa-Lys-Lys-Lys,
Xaa-Xaa-Lys-Xaa-Lys-Lys, Xaa-Xaa-Lys-Lys-Xaa-Lys, Xaa-Xaa-Lys-Lys-Lys-
Xaa, Xaa-Lys-Xaa-Xaa-Lys-Lys, Xaa-Lys-Xaa-Lys-Xaa-Lys, Xaa-Lys-Xaa-Lys-
Lys-Xaa, Xaa-Lys-Lys-Xaa-Xaa-Lys, Xaa-Lys-Lys-Xaa-Lys-Xaa, Xaa-Lys-Lys-
Lys-Xaa-Xaa, Lys-Lys-Lys-Xaa-Xaa-Xaa, Lys-Lys-Xaa-Lys-Xaa-Xaa, Lys-Lys-
Xaa-Xaa-Lys-Xaa, Lys-Lys-Xaa-Xaa-Xaa-Lys, Lys-Xaa-Lys-Lys-Xaa-Xaa, Lys-
Xaa-Lys-Xaa-Lys-Xaa, Lys-Xaa-Lys-Xaa-Xaa-Lys, Lys-Xaa-Xaa-Lys-Lys-Xaa,
Lys-Xaa-Xaa-Lys-Xaa-Lys, Lys-Xaa-Xaa-Xaa-Lys-Lys, Lys-Lys-Xaa-Xaa-Xaa-
Xaa, Lys-Xaa-Lys-Xaa-Xaa-Xaa, Lys-Xaa-Xaa-Lys-Xaa-Xaa-Lys, Lys-Xaa-Xaa-
Xaa-Lys-Xaa-Lys, Lys-Xaa-Xaa-Xaa-Xaa-Lys-Lys, Xaa-Lys-Lys-Xaa-Xaa-Xaa,
Xaa-Lys-Xaa-Lys-Xaa-Xaa, Xaa-Lys-Xaa-Xaa-Lys-Xaa, Xaa-Lys-Xaa-Xaa-Xaa-
Lys, Xaa-Xaa-Lys-Lys-Xaa-Xaa, Xaa-Xaa-Lys-Xaa-Lys-Xaa, Xaa-Xaa-Lys-Xaa-
Xaa-Lys, Xaa-Xaa-Xaa-Lys-Lys-Xaa, Xaa-Xaa-Xaa-Lys-Xaa-Lys, Xaa-Xaa-Xaa-
Xaa-Lys-Lys, Lys-Xaa-Xaa-Xaa-Xaa-Xaa, Xaa-Lys-Xaa-Xaa-Xaa-Xaa, Xaa-Xaa-
 
Lys-Xaa-Xaa-Xaa, Xaa-Xaa-Xaa-Lys-Xaa-Xaa, Xaa-Xaa-Xaa-Xaa-Lys-Xaa, Xaa-
Xaa-Xaa-Xaa-Xaa-Lys, Xaa-Xaa-Xaa-Xaa-Xaa-Xaa, Lys-Lys-Lys-Lys-Lys-Lys-
Lys, Xaa-Lys-Lys-Lys-Lys-Lys-Lys, Lys-Xaa-Lys-Lys-Lys-Lys-Lys, Lys-Lys-
Xaa-Lys-Lys-Lys-Lys, Lys-Lys-Lys-Xaa-Lys-Lys-Lys, Lys-Lys-Lys-Lys-Xaa-
Lys-Lys, Lys-Lys-Lys-Lys-Lys-Xaa-Lys, Lys-Lys-Lys-Lys-Lys-Lys-Xaa, Xaa-
Xaa-Lys-Lys-Lys-Lys-Lys, Xaa-Lys-Xaa-Lys-Lys-Lys-Lys, Xaa-Lys-Lys-Xaa-
Lys-Lys-Lys, Xaa-Lys-Lys-Lys-Xaa-Lys-Lys, Xaa-Lys-Lys-Lys-Lys-Xaa-Lys,
Lys-Xaa-Xaa-Lys-Lys-Lys-Lys, Lys-Xaa-Lys-Xaa-Lys-Lys-Lys, Lys-Xaa-Lys-
Lys-Xaa-Lys-Lys, Lys-Xaa-Lys-Lys-Lys-Xaa-Lys, Lys-Lys-Xaa-Xaa-Lys-Lys-
Lys, Lys-Lys-Xaa-Lys-Xaa-Lys-Lys, Lys-Lys-Xaa-Lys-Lys-Xaa-Lys, Lys-Lys-
Lys-Xaa-Xaa-Lys-Lys, Lys-Lys-Lys-Xaa-Lys-Xaa-Lys, Lys-Lys-Lys-Lys-Xaa-
Xaa-Lys, Xaa-Xaa-Xaa-Lys-Lys-Lys-Lys, Xaa-Xaa-Lys-Xaa-Lys-Lys-Lys, Xaa-
Xaa-Lys-Lys-Xaa-Lys-Lys, Xaa-Xaa-Lys-Lys-Lys-Xaa-Lys, Xaa-Lys-Xaa-Xaa-
Lys-Lys-Lys, Xaa-Lys-Xaa-Lys-Xaa-Lys-Lys, Xaa-Lys-Xaa-Lys-Lys-Xaa-Lys,
Xaa-Lys-Lys-Xaa-Xaa-Lys-Lys, Xaa-Lys-Lys-Xaa-Lys-Xaa-Lys, Xaa-Lys-Lys-
Lys-Xaa-Lys-Xaa, Xaa-Lys-Lys-Xaa-Lys-Lys-Xaa, Xaa-Lys-Xaa-Lys- Lys-Lys-
Xaa, Xaa-Lys-Lys-Lys-Xaa-Xaa-Lys, Lys-Xaa-Lys-Lys-Lys-Xaa-Xaa, Xaa-Lys-
Lys-Lys-Lys-Xaa-Xaa, Xaa-Lys-Lys-Lys-Xaa-Lys-Xaa, Xaa-Lys-Lys-Lys-Xaa-
Xaa-Lys, Lys-Lys-Lys-Lys-Xaa-Xaa-Xaa, Lys-Lys-Lys-Xaa-Xaa-Xaa-Lys, Lys-
Lys-Lys-Xaa-Lys-Xaa-Xaa, Lys-Lys-Xaa-Lys-Lys-Xaa-Xaa, Lys-Lys-Xaa-Xaa-
Lys-Xaa-Lys, Lys-Lys-Xaa-Xaa-Xaa-Lys-Lys, Lys-Lys-Xaa-Lys-Lys-Xaa-Xaa,
Lys-Xaa-Lys-Lys-Xaa-Xaa-Lys, Lys-Xaa-Lys-Xaa-Lys-Xaa-Lys, Lys-Xaa-Lys-
Xaa-Xaa-Lys-Lys, Lys-Xaa-Xaa-Lys-Lys-Xaa-Lys, Lys-Xaa-Xaa-Lys-Xaa-Lys-
Lys, Lys-Xaa-Xaa-Xaa-Lys-Lys-Lys, Lys-Lys-Xaa-Xaa-Xaa-Xaa-Lys, Lys-Xaa-
Lys-Xaa-Xaa-Xaa-Lys, Lys-Xaa-Xaa-Lys-Xaa-Xaa-Lys, Lys-Xaa-Xaa-Xaa-Lys-
Xaa-Lys, Lys-Xaa-Xaa-Xaa-Xaa-Lys-Lys, Xaa-Lys-Lys-Xaa-Xaa-Xaa-Lys, Xaa-
Lys-Xaa-Lys-Xaa-Xaa-Lys, Xaa-Lys-Xaa-Xaa-Lys-Xaa-Lys, Xaa-Lys-Xaa-Xaa-
Xaa-Lys-Lys, Xaa-Xaa-Lys-Lys-Xaa-Xaa-Lys, Xaa-Xaa-Lys-Xaa-Lys-Xaa-Lys,
Xaa-Xaa-Lys-Xaa-Xaa-Lys-Lys, Xaa-Xaa-Xaa-Lys-Lys-Xaa-Lys, Xaa-Xaa-Xaa-
 
Lys-Xaa-Lys-Lys, Xaa-Xaa-Xaa-Xaa-Lys-Lys-Lys, Lys-Xaa-Xaa-Xaa-Xaa-Xaa-
Lys, Xaa-Lys-Xaa-Xaa-Xaa-Xaa-Lys, Xaa-Xaa-Lys-Xaa-Xaa-Xaa-Lys, Xaa-Xaa-
Xaa-Lys-Xaa-Xaa-Lys, Xaa-Xaa-Xaa-Xaa-Lys-Xaa-Lys, Xaa-Xaa-Xaa-Xaa-Xaa-
Lys-Lys, Xaa-Xaa-Xaa-Xaa-Xaa-Xaa-Lys, Lys-Xaa-Xaa-Xaa-Xaa-Xaa-Xaa, Xaa-
Xaa-Xaa-Xaa-Xaa-Lys-Xaa, Xaa-Lys-Xaa-Xaa-Xaa-Xaa-Xaa, Xaa-Xaa-Lys-Xaa-
Xaa-Xaa, Xaa-Xaa-Xaa-Xaa-Lys-Xaa, Xaa-Xaa-Xaa-Xaa-Xaa-Xaa-Xaa,
 wherein each Xaa is independently selected from the group consisting of Ala, Leu, Ser, Thr, Tyr, Asn, Gln, Asp, Glu, Arg, His, Met, Orn, and amino acids of the formula I
-NH-C(R¹)(R²)-C(=O)- (I)
wherein R¹ and R² are selected from the group consisting of hydrogen, C₁₋₆-alkyl, phenyl, and phenyl-methyl, wherein C₁₋₆-alkyl is optionally substituted with from one to three substituents selected from halogen, hydroxy, amino, cyano, nitro, sulfono, and carboxy, and phenyl and phenyl-methyl is optionally substituted with from one to three substituents selected from C₁₋₆-alkyl, C₂₋₆-alkenyl, halogen, hydroxy, amino, cyano, nitro, sulfono, and carboxy, or R¹ and R² together with the carbon atom to which they are bound form a cyclopentyl, cyclohexyl, or cycloheptyl ring, e.g. 2,4-diaminobutanoic acid (Dbu) and 2,3-diaminopropanoic acid (Dpr).

7. The peptide conjugate according to any of the preceding claims, wherein said conjugate is selected from the group consisting of des Ser³⁹-exendin-4-Lys₆-NH₂, exendin-4-Lys₆-NH₂, des Pro³⁶-exendin-4-Lys₆-NH₂, des Ala³⁵-exendin-4-Lys₆-NH₂ and des Gly³⁴-exendin-4-Lys₆-NH₂, des Ser³⁹-(Lys⁴⁰ (palmitoyl))exendin-4-Lys₇-NH₂, des Gly³⁴-(Lys⁴⁰ (palmitoyl))exendin-4-Lys₇-NH₂, des Ala³⁵-(Lys⁴⁰ (palmitoyl))exendin-4-Lys₇-NH₂, des Pro³⁶-(Lys⁴⁰ (palmitoyl))exendin-4-Lys₇-NH₂ and Lys⁴⁰ (palmitoyl)exendin-4-Lys₇-NH₂.

8. A pharmaceutical composition comprising a pharmaceutically active peptide conjugate as defined in any of the claims 5-7, and a pharmaceutical acceptable carrier.

9. Use of a pharmaceutically active peptide conjugate of claims 5-7 for the manufacture of a pharmaceutical composition for use in treatment of diabetes type I or type 2, obesity, eating disorders, hyperglycemia, metabolic disorders and insulin resistance syndrome.


### Annex to the application documents - subsequently filed sequences listing

SEQUENCE LISTING

<110> ZEALAND PHARMACEUTICALS A/S

<120> NOVEL PEPTIDES THAT LOWER BLOOD GLUCOSE LEVELS

<130> 23488 EP1

<140> EP 99610043.4
<141> 1999-08-09

<150> US 60/143,591
<151> 1999-07-12

<160> 113

<170> PatentIn Ver. 2.1

<210> 1
<211> 4
<212> PRT
<213> Artificial Sequence

<220>
<223> Description of Artificial Sequence: synthetic peptide
      sequence

<400> 1
Lys Lys Lys Lys
  1


<210> 2
<211> 5
<212> PRT
<213> Artificial Sequence

<220>
<223> Description of Artificial Sequence: synthetic peptide
      sequence

<400> 2
Lys Lys Lys Lys Lys
  1               5


<210> 3
<211> 5
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,

Glu, Arg, His, Met, Orn, Dbu or Dpr

<400> 3
Xaa Lys Lys Lys Lys
1               5


<210> 4
<211> 5
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 4
Lys Xaa Lys Lys Lys
1               5


<210> 5
<211> 5
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 5
Lys Lys Xaa Lys Lys
1               5


<210> 6
<211> 5
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 6
Lys Lys Lys Xaa Lys
  1               5


<210> 7
<211> 5
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 7
Lys Lys Lys Lys Xaa
  1               5


<210> 8
<211> 6
<212> PRT
<213> Artificial Sequence

<220>
<223> Description of Artificial Sequence: synthetic peptide
      sequence

<400> 8
Lys Lys Lys Lys Lys Lys
  1               5


<210> 9
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 9
Xaa Lys Lys Lys Lys Lys
  1               5

<210> 10
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 10
Lys Xaa Lys Lys Lys Lys
  1               5


<210> 11
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 11
Lys Lys Xaa Lys Lys Lys
  1               5


<210> 12
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 12
Lys Lys Lys Xaa Lys Lys
  1               5


<210> 13

<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 13
Lys Lys Lys Lys Xaa Lys
  1               5


<210> 14
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 14
Lys Lys Lys Lys Lys Xaa
  1               5


<210> 15
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 15
Xaa Xaa Lys Lys Lys Lys
  1               5


<210> 16
<211> 6

<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 16
Xaa Lys Xaa Lys Lys Lys
  1               5


<210> 17
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 17
Xaa Lys Lys Xaa Lys Lys
  1               5


<210> 18
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 18
Xaa Lys Lys Lys Xaa Lys
  1               5


<210> 19
<211> 6
<212> PRT

<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 19
Xaa Lys Lys Lys Lys Xaa
  1               5


<210> 20
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 20
Lys Xaa Xaa Lys Lys Lys
  1               5


<210> 21
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 21
Lys Xaa Lys Xaa Lys Lys
  1               5


<210> 22
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 22
Lys Xaa Lys Lys Xaa Lys
  1               5


<210> 23
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 23
Lys Xaa Lys Lys Lys Xaa
  1               5


<210> 24
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 24
Lys Lys Xaa Xaa Lys Lys
  1               5


<210> 25
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 25
Lys Lys Xaa Lys Xaa Lys
  1               5


<210> 26
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 26
Lys Lys Xaa Lys Lys Xaa
  1               5


<210> 27
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 27
Lys Lys Lys Xaa Xaa Lys
  1               5


<210> 28
<211> 6
<212> PRT
<213> Artificial Sequence

<220>

<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 28
Lys Lys Lys Xaa Lys Xaa
  1               5


<210> 29
<211> 6
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 29
Lys Lys Lys Lys Xaa Xaa
  1               5


<210> 30
<211> 7
<212> PRT
<213> Artificial Sequence

<220>
<223> Description of Artificial Sequence: synthetic peptide
      sequence

<400> 30
Lys Lys Lys Lys Lys Lys Lys
  1               5


<210> 31
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,

Glu, Arg, His, Met, Orn, Dbu or Dpr

<400> 31
Xaa Lys Lys Lys Lys Lys Lys
1               5


<210> 32
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 32
Lys Xaa Lys Lys Lys Lys Lys
1               5


<210> 33
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 33
Lys Lys Xaa Lys Lys Lys Lys
1               5


<210> 34
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 34
Lys Lys Lys Xaa Lys Lys Lys
  1               5


<210> 35
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 35
Lys Lys Lys Lys Xaa Lys Lys
  1               5


<210> 36
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 36
Lys Lys Lys Lys Lys Xaa Lys
  1               5


<210> 37
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 37
Lys Lys Lys Lys Lys Lys Xaa
  1               5


<210> 38
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 38
Xaa Xaa Lys Lys Lys Lys Lys
  1               5


<210> 39
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 39
Xaa Lys Xaa Lys Lys Lys Lys
  1               5


<210> 40
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 40

Xaa Lys Lys Xaa Lys Lys Lys
  1               5


<210> 41
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 41
Xaa Lys Lys Lys Xaa Lys Lys
  1               5


<210> 42
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 42
Xaa Lys Lys Lys Lys Xaa Lys
  1               5


<210> 43
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 43
Lys Xaa Xaa Lys Lys Lys Lys
  1               5


<210> 44
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 44
Lys Xaa Lys Xaa Lys Lys Lys
  1               5


<210> 45
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 45
Lys Xaa Lys Lys Xaa Lys Lys
  1               5


<210> 46
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 46

Lys Xaa Lys Lys Lys Xaa Lys
  1               5


<210> 47
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 47
Lys Lys Xaa Xaa Lys Lys Lys
  1               5


<210> 48
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 48
Lys Lys Xaa Lys Xaa Lys Lys
  1               5


<210> 49
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 49
Lys Lys Xaa Lys Lys Xaa Lys
1               5


<210> 50
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 50
Lys Lys Lys Xaa Xaa Lys Lys
1               5


<210> 51
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 51
Lys Lys Lys Xaa Lys Xaa Lys
1               5


<210> 52
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 52
Lys Lys Lys Lys Xaa Xaa Lys
  1               5


<210> 53
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 53
Xaa Xaa Xaa Lys Lys Lys Lys
  1               5


<210> 54
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 54
Xaa Xaa Lys Xaa Lys Lys Lys
  1               5


<210> 55
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 55

Xaa Xaa Lys Lys Xaa Lys Lys
  1               5


<210> 56
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 56
Xaa Xaa Lys Lys Lys Xaa Lys
  1               5


<210> 57
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 57
Xaa Lys Xaa Xaa Lys Lys Lys
  1               5


<210> 58
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 58
Xaa Lys Xaa Lys Xaa Lys Lys
  1               5


<210> 59
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 59
Xaa Lys Xaa Lys Lys Xaa Lys
  1               5


<210> 60
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 60
Xaa Lys Lys Xaa Xaa Lys Lys
  1               5


<210> 61
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 61

Xaa Lys Lys Xaa Lys Xaa Lys
  1               5


<210> 62
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 62
Xaa Lys Lys Lys Xaa Lys Xaa
  1               5


<210> 63
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 63
Xaa Lys Lys Xaa Lys Lys Xaa
  1               5


<210> 64
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 64
Xaa Lys Xaa Lys Lys Lys Xaa
1               5


<210> 65
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 65
Xaa Lys Lys Lys Xaa Xaa Lys
1               5


<210> 66
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 66
Lys Xaa Lys Lys Lys Xaa Xaa
1               5


<210> 67
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 67

Xaa Lys Lys Lys Lys Xaa Xaa
1               5

<210> 68
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 68
Xaa Lys Lys Lys Xaa Lys Xaa
1               5

<210> 69
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 69
Xaa Lys Lys Lys Xaa Xaa Lys
1               5

<210> 70
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 70
Lys Lys Lys Lys Xaa Xaa Xaa
1               5


<210> 71
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 71
Lys Lys Lys Xaa Xaa Xaa Lys
1               5


<210> 72
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 72
Lys Lys Lys Xaa Lys Xaa Xaa
1               5


<210> 73
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 73

Lys Lys Xaa Lys Lys Xaa Xaa
  1               5


<210> 74
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 74
Lys Lys Xaa Xaa Lys Xaa Lys
  1               5


<210> 75
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 75
Lys Lys Xaa Xaa Xaa Lys Lys
  1               5


<210> 76
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 76
Lys Lys Xaa Lys Lys Xaa Xaa
  1               5


<210> 77
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 77
Lys Xaa Lys Lys Xaa Xaa Lys
  1               5


<210> 78
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 78
Lys Xaa Lys Xaa Lys Xaa Lys
  1               5


<210> 79
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 79

Lys Xaa Lys Xaa Xaa Lys Lys
1               5


<210> 80
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 80
Lys Xaa Xaa Lys Lys Xaa Lys
1               5


<210> 81
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 81
Lys Xaa Xaa Lys Xaa Lys Lys
1               5


<210> 82
<211> 7
<212> PRT
<213> Artificial Sequence

<220>
<223> Description of Artificial Sequence: synthetic peptide
      sequence

<220>
<223> Xaa at various positions throughout the sequence
      may be Ala, Leu, Ser, Thr, Tyr, Asn, Gln, Asp,
      Glu, Arg, His, Met, Orn, Dbu or Dpr

<400> 82
Lys Xaa Xaa Xaa Lys Lys Lys
  1               5


<210> 83
<211> 6
<212> PRT
<213> Artificial Sequence

<220>
<223> Description of Artificial Sequence: synthetic peptide
      sequence

<400> 83
Lys Glu Lys Glu Lys Glu
  1               5


<210> 84
<211> 6
<212> PRT
<213> Artificial Sequence

<220>
<223> Description of Artificial Sequence: synthetic peptide
      sequence

<400> 84
Glu Lys Glu Lys Glu Lys
  1               5


<210> 85
<211> 6
<212> PRT
<213> Artificial Sequence

<220>
<223> Description of Artificial Sequence: synthetic peptide
      sequence

<400> 85
Lys Lys Lys Glu Glu Glu
  1               5


<210> 86
<211> 6
<212> PRT
<213> Artificial Sequence

<220>
<223> Description of Artificial Sequence: synthetic peptide
      sequence

<400> 86
Glu Glu Glu Lys Lys Lys
  1               5


<210> 87
<211> 30
<212> PRT
<213> Artificial Sequence

<220>
<223> Description of Artificial Sequence:
      Gly8-GLP-1-(7-36)(Human)-NH2

<400> 87
His Gly Glu Gly Thr Phe Thr Ser Asp Val Ser Ser Tyr Leu Glu Gly
  1               5                  10                  15

Gln Ala Ala Lys Glu Phe Ile Ala Trp Leu Val Lys Gly Arg
             20                  25                  30


<210> 88
<211> 36
<212> PRT
<213> Artificial Sequence

<220>
<223> Description of Artificial Sequence:
      Gly8-GLP-1-(7-36)(Human)-Lys6-NH2

<400> 88
His Gly Glu Gly Thr Phe Thr Ser Asp Val Ser Ser Tyr Leu Glu Gly
  1               5                  10                  15

Gln Ala Ala Lys Glu Phe Ile Ala Trp Leu Val Lys Gly Arg Lys Lys
             20                  25                  30

Lys Lys Lys Lys
         35


<210> 89
<211> 38
<212> PRT
<213> Artificial Sequence

<220>
<223> Description of Artificial Sequence:
      Gly8Lys37(palmitoyl)-GLP-1-(7-36)(Human)-Lys7-NH2

<220>
<221> MOD_RES
<222> (31)
<223> Lys(palmitoyl)

<400> 89
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Lys Lys
            20                  25                  30

Lys Lys Lys Lys Lys Lys
        35

<210> 90
<211> 36
<212> PRT
<213> Artificial Sequence

<220>
<223> Description of Artificial Sequence:
      Gly8Lys34(palmitoyl)-GLP-1-(7-36)(Human)-Lys6-NH2

<220>
<221> MOD_RES
<222> (28)
<223> Lys(palmitoyl)

<400> 90
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Lys Lys
            20                  25                  30

Lys Lys Lys Lys
        35

<210> 91
<211> 44
<212> PRT
<213> Artificial Sequence

<220>
<223> Description of Artificial Sequence:
      des Ser39-exendin-4-Lys6-NH2

<400> 91
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Lys Lys Lys Lys Lys Lys
        35                  40

<210> 92
<211> 45

<212> PRT
<213> Artificial Sequence

<220>
<223> Description of Artificial Sequence:
      exendin-4-Lys6-NH2

<400> 92
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Gly Ala Pro Pro Pro Ser Lys Lys Lys Lys Lys Lys
        35                  40                  45


<210> 93
<211> 44
<212> PRT
<213> Artificial Sequence

<220>
<223> Description of Artificial Sequence:
      des Pro36-exendin-4-Lys6-NH2

<400> 93
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Gly Ala Pro Pro Ser Lys Lys Lys Lys Lys Lys
        35                  40


<210> 94
<211> 44
<212> PRT
<213> Artificial Sequence

<220>
<223> Description of Artificial Sequence:
      des Ala35-exendin-4-Lys6-NH2

<400> 94
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Gly Pro Pro Pro Ser Lys Lys Lys Lys Lys Lys
        35                  40

<210> 95
<211> 44
<212> PRT
<213> Artificial Sequence

<220>
<223> Description of Artificial Sequence:
      des Gly34-exendin-4-Lys6-NH2

<400> 95
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Ala Pro Pro Pro Ser Lys Lys Lys Lys Lys Lys
         35                  40


<210> 96
<211> 46
<212> PRT
<213> Artificial Sequence

<220>
<223> Description of Artificial Sequence:
      des Ser39-(Lys40(palmitoyl))exendin-4-Lys7-NH2

<220>
<221> MOD_RES
<222> (39)
<223> Lys(palmitoyl)

<400> 96
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Gly Ala Pro Pro Pro Lys Lys Lys Lys Lys Lys Lys Lys
         35                  40                  45


<210> 97
<211> 46
<212> PRT
<213> Artificial Sequence

<220>
<223> Description of Artificial Sequence:
      des Gly34-(Lys40(palmitoyl))exendin-4-Lys7-NH2

<220>
<221> MOD_RES
<222> (40)
<223> Lys(palmitoyl)

<400> 97
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Ala Pro Pro Pro Ser Lys Lys Lys Lys Lys Lys Lys Lys
        35                  40                  45


<210> 98
<211> 46
<212> PRT
<213> Artificial Sequence

<220>
<223> Description of Artificial Sequence:
      des Ala35-(Lys40(palmitoyl))exendin-4-Lys7-NH2

<220>
<221> MOD_RES
<222> (39)
<223> Lys(palmitoyl)

<400> 98
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Pro Pro Pro Ser Lys Lys Lys Lys Lys Lys Lys Lys
        35                  40                  45


<210> 99
<211> 46
<212> PRT
<213> Artificial Sequence

<220>
<223> Description of Artificial Sequence:
      des Pro36-(Lys40(palmitoyl))exendin-4-Lys7-NH2

<220>
<221> MOD_RES
<222> (39)
<223> Lys(palmitoyl)

<400> 99
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Lys Lys Lys Lys Lys Lys Lys Lys
           35                  40                  45

<210> 100
<211> 47
<212> PRT
<213> Artificial Sequence

<220>
<223> Description of Artificial Sequence:
      Lys40(palmitoyl)exendin-4-Lys7-NH2

<220>
<221> MOD_RES
<222> (40)
<223> Lys(palmitoyl)

<400> 100
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Gly Ala Pro Pro Pro Ser Lys Lys Lys Lys Lys Lys Lys Lys
        35                  40                  45

<210> 101
<211> 38
<212> PRT
<213> Artificial Sequence

<220>
<223> Description of Artificial Sequence:
      des Pro36-exendin-4-NH2

<400> 101
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Gly Ala Pro Pro Ser
        35

<210> 102
<211> 39
<212> PRT
<213> Artificial Sequence

<220>
<223> Description of Artificial Sequence:
      exendin-4-NH2

<400> 102
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35


<210> 103
<211> 36
<212> PRT
<213> Artificial Sequence

<220>
<223> Description of Artificial Sequence:
      Gly8Lys26(palmitoyl)-GLP-1-(7-36)(Human)-Lys6-NH2

<220>
<221> MOD_RES
<222> (20)
<223> Lys(palmitoyl)

<400> 103
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Lys Lys
            20                  25                  30

Lys Lys Lys Lys
        35


<210> 104
<211> 38
<212> PRT
<213> Artificial Sequence

<220>
<223> Description of Artificial Sequence:
      des Ser39-exendin-4-NH2

<400> 104
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro
        35


<210> 105
<211> 38

<212> PRT
<213> Artificial Sequence

<220>
<223> Description of Artificial Sequence:
      des Ala35-exendin-4-NH2

<400> 105
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Gly Pro Pro Pro Ser
         35


<210> 106
<211> 38
<212> PRT
<213> Artificial Sequence

<220>
<223> Description of Artificial Sequence:
      des Gly34-exendin-4-NH2

<400> 106
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Ala Pro Pro Pro Ser
         35


<210> 107
<211> 39
<212> PRT
<213> Artificial Sequence

<220>
<223> Description of Artificial Sequence:
      des Ser39-(Lys40 (palmitoyl))exendin-4-NH2

<220>
<221> MOD_RES
<222> (39)
<223> Lys(palmitoyl)

<400> 107
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Gly Ala Pro Pro Pro Lys
        35

<210> 108
<211> 39
<212> PRT
<213> Artificial Sequence

<220>
<223> Description of Artificial Sequence:
      des Gly34-(Lys40 (palmitoyl))exendin-4-NH2

<220>
<221> MOD_RES
<222> (39)
<223> Lys(palmitoyl)

<400> 108
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Ala Pro Pro Pro Ser Lys
        35

<210> 109
<211> 39
<212> PRT
<213> Artificial Sequence

<220>
<223> Description of Artificial Sequence:
      des Ala35-(Lys40 (palmitoyl))exendin-4-NH2

<220>
<221> MOD_RES
<222> (39)
<223> Lys(palmitoyl)

<400> 109
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Gly Pro Pro Pro Ser Lys
        35

<210> 110
<211> 39
<212> PRT

<213> Artificial Sequence

<220>
<223> Description of Artificial Sequence:
      des Pro36-(Lys40 (palmitoyl))exendin-4-NH2

<220>
<221> MOD_RES
<222> (39)
<223> Lys(palmitoyl)

<400> 110
His Gly Glu Gly Thr Phe Thr Ser Asp Leu Ser Lys Gln Met Glu Glu
  1               5                  10                  15

Glu Ala Val Arg Leu Phe Ile Glu Trp Leu Lys Asn Gly Gly Pro Ser
             20                  25                  30

Ser Gly Ala Pro Pro Ser Lys
         35


<210> 111
<211> 31
<212> PRT
<213> Artificial Sequence

<220>
<223> Description of Artificial Sequence:
      Gly8Lys37N-palmitoyl-GLP-1 (7-36)

<220>
<221> MOD_RES
<222> (31)
<223> Lys(N-palmitoyl)

<400> 111
His Gly Glu Gly Thr Phe Thr Ser Asp Val Ser Ser Tyr Leu Glu Gly
  1               5                  10                  15

Gln Ala Ala Lys Glu Phe Ile Ala Trp Leu Val Lys Gly Arg Lys
             20                  25                  30


<210> 112
<211> 30
<212> PRT
<213> Artificial Sequence

<220>
<223> Description of Artificial Sequence:
      Gly8Lys34N-palmitoyl-GLP-1 (7-36)

<220>
<221> MOD_RES
<222> (28)
<223> Lys(N-palmitoyl)

<400> 112
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210> 113
<211> 30
<212> PRT
<213> Artificial Sequence

<220>
<223> Description of Artificial Sequence:
      Gly8Lys26N-palmitoyl-GLP-1 (7-36)

<220>
<221> MOD_RES
<222> (20)
<223> Lys(N-palmitoyl)

<400> 113
His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30